# EUROPEAN PATENT SPECIFICATION

(11) **EP 2 914 612 B1**
(45) Date of publication and mention of the grant of the patent: **14.11.2018**
(21) Application number: 13794828.7
(22) Date of filing: 28.10.2013
(51) Int. Cl.: C07K 1/34

(54) **PURIFICATION OF POLYPEPTIDES USING DUAL STAGE TANGENTIAL-FLOW ULTRAFILTRATION**
REINIGUNG VON POLYPEPTIDEN MIT ZWEISTUFIGER TANGENTIALFLUSSULTRAFILTRIERUNG
PURIFICATION DE POLYPEPTIDES AU MOYEN DE L'ULTRAFILTRATION D'ÉCOULEMENT TANGENTIEL À DOUBLE ÉTAGE

(30) Priority: 30.10.2012 EP 12190682
(43) Date of publication of application: 09.09.2015
(73) Proprietor: F. Hoffmann-La Roche AG, 4070 Basel (CH)
(72) Inventor: BECKER, Peter, 82377 Penzberg (DE); NEUMANN, Sebastian, 79576 Weil am Rhein (DE)
(74) Representative: Vossius & Partner Patentanwälte Rechtsanwälte mbB
(86) International application number: PCT/EP2013/072511
(87) International publication number: WO 2014/067898

(56) References cited:
- WO-A1-2004/076695
- WO-A2-2007/106078
- DE-A1- 3 716 363
- PARKER M H ET AL: "Purification and characterization of a recombinant version of human alpha-fetoprotein expressed in the milk of transgenic goats", PROTEIN EXPRESSION AND PURIFICATION, ACADEMIC PRESS, SAN DIEGO, CA, vol. 38, no. 2, 1 December 2004 (2004-12-01), pages 177-183, XP004649872, ISSN: 1046-5928, DOI: 10.1016/J.PEP.2004.07.007

## Description

### FIELD OF THE INVENTION

The present invention is directed to methods for the separation of a molecule of interest from a solution containing the molecule using dual stage tangential-flow ultrafiltration ("TFF"). In particular, the methods of the invention are directed to the processing of crude feed streams such as conditioned cell culture supernatant to dramatically reduce contaminant and/or impurity levels prior to subsequent, *i.e.,* downstream, refining unit operations. The methods of the invention may be used in the processing of a crude feed stream from biological production systems such as fermentation or other cell culture process, and may further eliminate the need for time consuming impurity precipitation (*e.g*., pH driven) and/or precipitate filtration processes prior to downstream processes that are sensitive to high impurity loads such as chromatographic unit operations. The disclosed dual stage TFF process combines at least two TFF unit operations that may be advantageously conducted at a pH that corresponds to or is about that of the pH of the feed stream, *e.g.,* a cell culture supernatant, typically a pH of 7.5 ± 1.0. The use of the TFF unit operations to supplement, improve or replace traditional processes for purification of proteins of interest for a feed stream may represent significant savings in both direct and indirect processing costs, For example, in addition to indirect savings by eliminating precipitation and precipitate filtration processes, the reduction in impurity loads effected by the dual stage TFF unit operations may result in indirect savings by improving downstream column performance, e.g., chromatographic separation, dynamic binding capacity, operational lifetime and/or a reduction of the required column size. In particular embodiments, the methods of the invention are used in processes for the purification of immunoglobulin molecules, e.g., antibodies, which processes are devoid of affinity purification steps, *e.g.*, protein A affinity chromatography purification.

### BACKGROUND OF THE INVENTION

The present invention is directed to methods of separation and/or purification of molecules of interest, in particular, biomolecules (*e.g.*, proteins), from crude and/or production solutions containing the molecules. As the field of biotechnology advances, the economic processing of large quantities of biomolecules from crude production solutions has become increasingly relevant. In particular, the economics of production and processing can play a determinative role in decisions relating to the development and manufacture of biomolecules and/or pharmaceuticals, including enzymes, antibodies and other specialty proteins and peptides. For the production of the majority of such biomolecules (*e.g*., proteins, polypeptides, antibodies), the use of recombinant DNA techniques has become the production method of choice. Using such methods, large quantities of desired biomolecules can be expressed in a variety of biological systems. Such systems including cell based systems (such as bacteria, yeast, insect and mammalian cell cultures (*e.g*., having endogenous expression of the protein of interest or transgenic cell cultures)), transgenic animals and transgenic plants. The biomolecular pathways of the cell culture systems or *in vivo* systems have also been reduced to essential components for the implementation of *in vitro* production systems. However, with these production systems, the biopharmaceutical industry faces the parallel challenge of developing improved or novel systems for the recovery of the desired biomolecule (*e.g.*, protein) from the crude production solutions or production materials. The crude production solutions include conditioned cell culture media, clarified homogenized/lysed conditioned cell cultures, body fluids from transgenic animals (e.g., blood, serum, milk and urine) and *in vitro* production solutions that include starting materials and components in addition to the biomolecule of choice. The recovery methods must not only result in the recovered product meeting governmental purity and safety standards, but must also be economically feasible.

Purification processes particularly advantageous for the isolation of biomolecules from crude productions solutions typically include affinity-based chromatography processes and/or unit operations. Affinity-based chromatography separates biomolecules on the basis of specific interaction of the biomolecule of interest with a binding moiety that is immobilized to a solid substrate such as a matrix or a gel. Thus, the biomolecule of interest becomes bound to the substrate (typically contained within a column), while contaminants and other unwanted components of the feed stream flow through the system. The biomolecule is then eluted from the binding moiety and/or column and collected. Accordingly, affinity chromatography columns are highly specific and can yield very pure products. With respect to the purification of antibodies, e.g., from cell culture media, the binding moiety most often used for affinity chromatography is Protein A. Protein A affinity chromatography is highly selective for antibodies and can lead to a product purity of greater than 95% starting from an input sample such as conditioned cell culture media.

Although affinity chromatography is a powerful capturing and purification step, the use of bioaffinity moieties (also known in the art as bioaffinity ligands) that bind to the desired biomolecules is also associated with high processing costs. For example, bioaffinity ligands (which are normally biomolecules themselves), *e.g*., Protein A, cost 7 to 15 times as much as other chromatography materials (see, Gottschalk, Process Scale Purification of Antibodies (John Wiley & Sons, 1st ed., 2009), chapter 5.3.1). Additionally, bioaffinity ligands are subject to leaching from the substrate and column. Not only does this significantly shorten the lifespan of the column relative to one using, *e.g*., filtration membranes, the leached ligands have the potential to denaturate and can induce aggregate formation in the molecule of interest. Additionally, many bioaffinity ligands such as Protein A is/would be considered a toxin, requiring the product stream to be continually monitored for its presence. Thus, the use of bioaffinity ligands represents a significant outlay of not only direct costs, such as for materials of the column itself and increased replacement schedules, but also of indirect costs associated with process upkeep, monitoring and potential additional unit operations (*e.g*., requirements for precipitate/aggregate filtration and/or reprocessing). Therefore bioaffinity columns, their upkeep and their monitoring can represent the most significant costs in any purification scheme and can be difficult to integrate in a cost effective manner into industrial operations.

In view of the high costs associated with the use of bioaffinity ligands, a number of alternatives have been explored to supplement or improve affinity chromatography methods, including synthetic affinity ligands and mixed-mode resins (see, Gottschalk 2009, chapter 5.3.2). Additionally, processing/purification methods completely eliminating the affinity unit operation have also been investigated. Such methods include filtration, ion exchange chromatography, hydrophobic chromatography, gel filtration chromatography and combinations thereof. For example, a systematic evaluation of a three step chromatography process for the purification of an antibody not including Protein A chromatography has been published by Follmann et al., J. Chromatog. 1024(2004), 79-85.

Some of the most extensively investigated biomolecular purification processes for supplementation or replacement of affinity-based processing are those comprising ion exchange chromatography processes. In particular, cation exchange chromatography ("CEX") has been investigated in a number of protein purification schemes, both in conjunction with and as replacement for affinity-based unit operations (see, *e.g.*, Arunakumari et al., Adv. Process Chromatog. (Supp. BioPharm Int.) (2007),36-40; Arunakumari et al, BioPharm. Int. Supp. March 2, 2009; Wang et al., BioPharm. Int. Supp. March 2, 2008; Gottschalk 2009, chapter 5.3.3; Cacciuttolo, Pharmaceutical Process Scale-Up (CRC Press, 2nd ed. (2006), chapter 5; Morrow, Gen. Eng. Biotech. News 28(2008; available online only)). Despite its promise, ion exchange chromatography has not completely replaced affinity chromatography, but has been adapted to serve as an additional purification process in conjunction with affinity chromatography in common industrial purification processes. However, in chromatography processes, the composition of the loading material (*e.g.,* the product stream introduced into the process/unit operation/column) is critical to the proper functioning of the column. Therefore, in purification methods having chromatography processes/unit operations, processing of the load material prior to the chromatographic process is an essential first step. For example, prior to the chromatographic unit operation, the feed stream (containing the molecule of interest) must normally be adjusted to a suitable pH, buffering and conductivity depending on the particular isoelectric point of the molecule of interest and the particular column parameters. Thus, it is not only the particular chromatographic process but also the feed stream composition that detennines what modifications of the feed stream are necessary. Variations in the feed stream can thus require significant modification of unit operations and feed stream processing upstream of the ion chromatography process.

The processing/adjustment of feed stream parameters is often performed using diafiltration/tangential flow filtration ("TFF"). During this processing step, the feed stream solvent is gradually exchanged by diafiltration during TFF with a buffer appropriate for the particular chromatographic operation. For example, with respect to the purification of antibodies using CEX, most processes adjust the feed stream prior to CEX to a pH of about 5 to 7 with low conductivity because many antibodies exhibit isoelectric points greater than 7. However, where the feed stream is conditioned cell media (*e.g*., supernatant or homogenized cell culture), adjusting the feed stream pH below neutral during the buffer exchange often results in the precipitation of contaminants such as host cell DNA ("HCDNA") and host cell proteins ("HCP") (see, e.g., Gottschalk 2009, chapter 5.3.3; Wang et al, 2008).

The precipitation event is of significant economic importance not only because it forces the addition of at least one further unit operation (to remove the precipitates), but also because the precipitation processes can negatively impact the buffer exchange unit operation itself (*e.g.,* during operation, the diafiltration/TFF column can become clogged with precipitate). Thus, the processing of proteins from recombinant cell cultures using chromatographic processes normally requires the use of costly unit operations such as precipitation tanks and/or filtration units (see, *e.g.*, Wang et al, 2009).

Therefore, despite the promise of ion exchange chromatography, few have proven feasible in the industrial setting to completely replace affinity-based unit operations, and most commercial production processes of biopharmaceuticals retain an affinity purification step in conjunction with an ion-exchange unit operation. However, as detailed above, these multiple unit operations are rarely compatible, often requiring significant modification of buffer parameters that add cost and time to purification schemes. Accordingly, there is a need for the further development of purification processes for recombinantly produced biomolecules, *e.g*., proteins, that allow the purification potential offered by non-affinity based processes to be better realized in a more consistent manner. In particular, there is a need for processing steps that are compatible with a wide range of feed streams; that are able to significantly reduce contaminant loads, in particular, contaminant host cell proteins (HCP) and/or host cell DNA (HCDNA); and that are compatible with other unit operations such as affinity-based chromatography and with downstream processes such as ion exchange chromatography.

### SUMMARY OF THE INVENTION

The present invention is directed to methods for the separation of a molecule of interest from a cell culture solution containing the molecule using dual-stage tangential-flow ultrafiltration ("TFF"). The dual-stage TFF methods described herein substantially reduce contaminant and/or impurity levels in a product feed stream, and are particularly useful as upstream processes implemented prior to standard purification and isolation unit operations that may be used to bring the product stream to final formulation and/or purity, for example ion exchange chromatography. The dual-stage TFF methods described herein are particularly envisioned to supplement and, thus, be compatible with standard purification methods such as affinity-based chromatography unit operations and ion-exchange chromatography. However, the invention also encompasses methods wherein the disclosed dual-stage TFF methods replace affinity-based chromatography unit operations in the production and/or purification of a molecule of interest. The methods of the invention may be used in the processing of a crude feed stream (*i.e.,* a cell culture solution) from any cell culture systems including fermentations or other cell culture process to substantially reduce impurities or contaminants such as host cell proteins (HCP) and host cell DNA (HCDNA), which output streams may then be processed using standard purification methods, wither with or without affinity-based chromatography unit operations. The substantial reduction in contaminants and/or impurities of the feed stream (*i.e.,* from the cell culture supernatant) offered by the use of the disclosed dual-stage TFF methods eliminates the need for standard purification unit operations such as, for example, pH driven impurity precipitation and/or precipitate filtration (usually required prior to ion exchange chromatography due to the required changes in buffer composition) and/or may improve the efficiency of or even eliminate costly affinity-based unit operations. The dual-stage TFF methods disclosed herein are particularly useful as upstream processes implemented prior unit operations that are particularly sensitive to impurity loads such as chromatographic unit operations. For example, with respect to the use of downstream chromatographic unit operations, *e.g*., columns, the substantial reduction in contaminate load effected by the methods of the invention may act to improve chromatographic separation, improve dynamic binding capacity, to increase operational lifetime and/or to reduce the required column size, all of which significantly reduce both direct and indirect costs associated with the production, processing and purification of the molecule of interest.

In particular, the methods of the present invention provide for the separation of a molecule of interest from a cell culture solution containing the molecule using dual-stage TFF, which methods substantially reduce contaminant and/or impurity levels. The present inventors have discovered that a dual stage TFF process combining at least two TFF unit operations (one processing the molecule of interest in the retentate and one processing the molecule of interest in the permeate) can reduce contaminant and/or impurity levels in the product stream (*i.e*., the solution containing the molecule of interest during processing) to such a surprising extent that downstream contaminant removal via standard processing (such as precipitation (in particular, mediated via pH adjustment), precipitate filtration and affinity-based separation) can be minimized or eliminated. Accordingly, the methods of the present invention are particularly useful in processes comprising downstream chromatographic processes, which may be particularly sensitive to the composition of the product stream. In this respect, the dual stage TFF methods of the present invention effect not only the substantial reduction in contaminant and/or impurity levels of the product stream, but may also be concurrently used to adjust the product stream parameters, *e.g*., pH and/or conductivity, such that they are compatible with downstream unit operations. In this manner, the methods of the invention are highly complimentary to downstream chromatographic processes such as cation exchange chromatography ("CEX") and anion exchange chromatography ("AEX").

The dual stage TFF processes are particularly suited to process cell culture solutions prior to downstream, *e.g*., chromatographic, purification and isolation processes that are used to bring the product stream to final formulation and/or purity. The TFF process disclosed herein may be advantageously conducted at a pH that corresponds to or is about that of a typical crude product stream/cell culture solution from a biological process (*e.g*., conditioned cell culture media, cell culture lysates, conditioned fermentation media, etc.). Typically, the TFF methods disclosed herein are conducted at a pH of 7.5 ± 1.0 or 7.5 ± 0.5. The dual stage TFF methods disclosed herein can be combined with downstream standard purification processes known in the art such as affinity purification or, in particular, with downstream purification processes that are not based on affinity isolation, *e.g*., chromatography processes. In particular, the methods disclosed herein reduce contaminant levels such that the pH of the product stream can be modified downstream, *e.g.*, decreased to at least about 5, without precipitation of any remaining contaminants such host cell proteins (HCP) and/or host cell DNA (HCDNA) that may be in the product stream.

The methods of the invention can be used for any species of molecule, and are exemplified herein in a preferred embodiment for the separation of a protein from a solution containing the protein. The dual stage TFF methods disclosed herein are robust and scalable processes generically applicable to proteins over a wide range of sizes and molecular weights (*e.g*., polypeptides, peptides, immunoglobulins, antibodies, enzymes). The methods disclosed herein generically separate the protein of interest from the solution based on size. In other words, the methods of the invention are not based on any specific affinity or affinity-based purification process, and therefore allow the economic and accelerated processing of a variety of proteins from a variety of cell culture solution feed streams. Thus, the methods of the invention may result in savings of indirect costs associated with protein processing, production and/or purification, for example, saving costs associated with feed stream analysis, or pre-processing of the feed stream to ensure compatibility with existing systems.

The methods of the invention are particularly suited for the separation of a protein of interest from a feed stream that is a cell culture solution. As used herein, the term cell culture solution and analogous terms refer to any solution of a biological process or system expected to contain the protein of interest. Therefore, the term cell culture solution may denote the entire content of the vessel wherein fermentation of the cell culture, *i.e.,* production of the heterologous or endogenous protein, has been performed. The cell culture solution according to the invention may comprise not only the protein of interest, but also other proteins or protein fragments (*e.g.*, endogenously or heterologously produced by the cell culture or otherwise present in the medium (*e.g.,* as supplied)); cells of the cell culture; cell fragments; and/or other constituents supplied with the nutrient medium to support cell growth and protein production or constituents produced by the host cell during cultivation. Thus, the cell culture solutions of the invention include but are not limited to conditioned cell culture supernatants; clarified conditioned cell culture supernatants (*e.g.*, conditioned cell culture supernatant having particulate matter removed by standard methods known in the art); and clarified, homogenized/lysed cell cultures. In all aspects, the cell culture solution is understood to contain the molecule of interest; that is, it is understood to be a conditioned cell culture solution, *e.g.*, a conditioned cell culture supernatant. In preferred embodiments of the invention, the feed stream of the dual stage TFF is a cell culture supernatant from a fermentation reaction.

As is recognized in the art and as disclosed herein, the cell culture solution is generally clarified or sterilized by filtering through a filter having a pore size of between 0.1 µm to 0.45 µm, preferably a filter having a pore size of 0.2 µm or 0.22 µm, as a first stage in any processing scheme. Accordingly, in certain embodiments, the dual stage TFF methods disclosed herein comprise filtration of the cell culture solution through a suitable filter for sterilization, *e.g*., having a pore size of between 0.1 µm and 0.45 µm, preferably a pore size of 0.2 µm or 0.22 µm, upstream of a dual stage TFF method disclosed herein (*i.e.,* upstream of said at least two TFF unit operations). The use of other sizes of filters outside the ranges disclosed herein, whether preferred or not, for sterilization of the cell culture solution as is known or understood in the art and is also encompassed by the present invention. The present invention does not encompass the processing of milk, a fractionated solution from milk and/or product from milk such as whey protein isolate. Thus, for all embodiments disclosed herein, the feed stream and/or product stream containing the molecules of interest, *e.g.*, a protein, is not milk, a fractionated solution from milk and/or product from milk such as whey protein isolate.

The present invention provides a method of separating a protein of interest from a cell culture solution containing said protein using dual stage TFF, wherein the dual stage TFF comprises a first TFF unit operation and a second TFF unit operation, and wherein
(i) said first TFF unit operation filters a first product stream containing said protein such that said protein is recovered in the retentate of the first TFF unit operation; and
(ii) said second TFF unit operation filters a second product stream containing said protein such that said protein is recovered in the permeate of the second TFF unit operation.

As disclosed herein, the TFF unit operations of the invention comprise the use of ultrafiltration membranes. Thus, as defined herein, the abbreviation, "TFF", when used in the context of the invention is understood to reference tangential-flow ultrafiltration as that unit operation is commonly understood in the art. The cut-off values of the membranes of the at least two TFF unit operations (*i.e.,* of the first and second TFF unit operations) are selected such that the molecule of interest, e.g., protein, (i) does not pass through the membrane of the first TFF unit operation (*i.e.,* is recovered in the retentate of the first TFF unit operation), and (ii) does pass through the membrane of the second TFF unit operation (*i.e.,* is recovered in the permeate of the second TFF unit operation). Therefore, the invention may be further characterized as a method of separating a protein of interest from a solution containing the protein using dual stage TFF, wherein the dual stage TFF comprises a first TFF unit operation and a second TFF unit operation, and wherein
(i) the first TFF unit operation filters a first product stream containing the protein using a first ultrafiltration membrane having a cut-off value such that the protein of interest is recovered in the retentate of the first TFF unit operation; and
(ii) the second TFF unit operation filters a second product stream containing the protein through a second ultrafiltration membrane having a cut-off value such that the protein of interest is recovered in the permeate of the second TFF unit operation.

As defined herein, the ultrafiltration membrane of the first TFF unit operation is selected such that the molecule of interest does not pass through the membrane during the first TFF operation (*i.e.,* such that the protein is recovered in the retentate of the first TFF unit operation), and the ultrafiltration membrane of the second TFF unit operation is selected such that the molecule of interest does pass through the membrane during the second TFF operation (*i.e.,* such that the protein is recovered in the permeate of the second TFF unit operation). Normally, filtration membranes are classified by pore size or cut-off values, which define membrane performance based on the membrane's maximum pore size and/or the maximum molecular weight of a freely permeable molecule in kD; thus, molecules of sizes above the cut-off value of maximum pore size and/or above the maximum molecular weight should not pass through the membrane. However, as recognized in the art, because filtration membrane performance can also depend on factors other than strict molecular size, *e.g.*, molecular charge, the present disclosure does not limit the criteria by which the membranes may be selected provided that the molecule of interest, *e.g.*, protein does not pass through the first membrane in the first TFF unit operation and does pass through the second membrane of the second TFF unit operation. Therefore, the cut-off values of the membranes used according to the dual stage TFF methods disclosed herein may be selected based on membrane performance rather than solely based on an asserted cut-off value or pore size (*e.g*., according to a manufacturer's specifications). Methods to determine membrane performance and, specifically, to determine the permeability of a membrane to a molecule of interest, *e.g.*, protein, are well known and routinely implemented in the art.

In connection with the TFF unit operations of the invention, *e.g.*, the first TFF unit operation as disclosed in connection with the present invention, the phrases "does not pass through the membrane" and "is in the retentate of the TFF unit operation" are equivalent and indicate that the membrane of the TFF unit operation is essentially impermeable to the molecule of interest, *e.g.* a protein. Further, because membrane performance can vary in connection with biological solutions and molecules (*e.g.*, proteins) as recognized in the art, it is understood that the phrases "does not pass through the membrane", "is in the retentate of the TFF unit operation" and/or "essentially impermeable", as these terms and phrases are used throughout this disclosure, are not to be interpreted as absolute expressions. Rather, as used herein and in accordance with the understanding in the art, the phrases are used with an appreciation that the first filtration membrane may exhibit "breakthrough" (*i.e.,* allow minimal amounts of the molecule of interest to permeate through the membrane), but that at least 90% of the amount of the molecule of interest applied to the first TFF unit operation is or can be recovered in the retentate.

Similarly, in connection with the TFF unit operations of the invention, e.g., the second TFF unit operation as disclosed in connection with the present invention, the phrases "does pass through the membrane" and "is in the permeate of the TFF unit operation" are equivalent and indicate that the membrane of the TFF unit operation is essentially freely permeable to the molecule of interest, *e.g.* a protein. Further, because membrane performance can vary in connection with biological solutions and molecules (*e.g.*, proteins) as recognized in the art, it is understood that that the phrases "does pass through the membrane", "is in the permeate of the TFF unit operation" and/or "essentially freely permeable", as these terms and phrases are used throughout this disclosure, are not to be interpreted as absolute expressions. Rather, as used herein and in accordance with the understanding in the art, the phrases are used with an appreciation that the filtration membrane may not be freely permeable to 100% of the molecules of interest applied to/through the filter and that the membrane may exhibit impermeability to some minor percentage of the applied load of the molecule of interest. Therefore, as used herein, the phrases indicate that at least 90% of the amount of the molecule of interest applied to the second TFF unit operation is or can be recovered in the permeate.

Accordingly, the dual-stage TFF methods disclosed herein typically encompass the use of at least two ultrafiltration membranes, each having a different cut-off value, pore size and/or permeability measurement. The ultrafiltration membrane of the first TFF unit operation is selected such that the molecule of interest does not pass through the membrane during operation and can be recovered in the retentate of the operation. Typically, this is achieved by selecting the first ultrafiltration membrane (*i.e.,* the membrane of the first TFF unit operation) as having a cut-off value that is less than the molecular weight of the molecule of interest. However, as recognized in the art, membrane performance can depend on other factors beyond molecular size; thus, membranes having specified cut-off values may nevertheless be extremely efficient at blocking the passage of molecules with a size below the stated cut off value. Therefore, membranes with cut-off values greater than the molecular weight of the molecule of interest may be used in the first TFF unit operation provided that the molecule of interest does not pass through the membrane during the first TFF unit operation. As such, in accordance with the invention, the particular membrane selected for use in the first TFF unit operation is not in any other manner limited and may be selected based on manufacturing ease or commercial availability provided it meets the other criteria set forth herein. In embodiments of the disclosed invention, the cut-off value of the filter membrane of the first TFF unit operation may be 1.5, 1, ½, 1/3, 1/5, 1/10, or 1/15 times the molecular weight of the molecule of interest, e.g., protein, provided that the molecule of interest does not pass through the membrane during the first TFF unit operation. In other embodiments, the cut-off value of the filter membrane of the first TFF unit operation is less than 1/15 of the molecular weight of the molecule of interest. In specific embodiments, the cut-off value of the filter membrane of the first TFF unit operation as disclosed herein is no greater than about one-half of the molecular weight of the molecule of interest, *e.g.*, protein. In other words, in these specific embodiments, the cut-off value of the filter membrane of the first TFF unit operation as disclosed herein is 0.5 times the molecular weight of the molecule of interest or less. Selection of the particular membrane may also depend on additional parameters such as availability (*e.g.*, whether commercially available or whether available by self-manufacture) or performance (*e.g.*, performance in separating the molecule of interest from a specific known contaminant/impurity).
The present invention also provides that the ultrafiltration membrane of the second TFF unit operation is selected such that the molecule of interest does pass through the membrane during operation and can be recovered in the permeate of the unit operation. Typically, this is achieved by selecting the second ultrafiltration membrane (*i.e.,* the membrane of the second TFF unit operation) as having a cut-off value that is greater than the molecular weight of the molecule of interest, but below a maximum of 1000 kD (which is the maximum size of a molecule for "ultrafiltration" as explained herein). Accordingly, the particular membrane selected for use in the second TFF unit operation is not in any other manner limited and may be selected based on manufacturing ease or commercial availability provided it meets the criteria set forth herein, namely that the molecule of interest passes through the membrane in the second TFF unit operation, which second membrane has a maximum cut-off value of 1000 kD. In embodiments of the disclosed invention, the cut-off value of the filter membrane of the second TFF unit operation may be 1, 1.5, 2, 3, 4, 5, 10, 15, 20, 25, 50 or 100 times the molecular weight of the molecule of interest, *e.g.*, protein, provided that the molecule of interest does pass through the filter membrane during the second TFF unit operation and provided that the cut-off value does not exceed 1000 kD.
Therefore, the invention also provides a method of separating a protein of interest from a solution containing the protein using dual stage TFF, wherein the dual stage TFF comprises a first TFF unit operation and a second TFF unit operation, and wherein
(i) the first TFF unit operation filters a first product stream containing the protein using a first ultrafiltration membrane having a cut-off value such that the protein of interest is recovered in the retentate of the first TFF unit operation, and/or wherein the cut-off value of the first ultrafiltration membrane is 0.5 times the molecular weight of the protein; and
(ii) the second TFF unit operation filters a second protduct stream containing the protein through a second ultrafiltration membrane having a cut-off value such that the protein of interest is recovered in the permeate of the second TFF unit operation, wherein the cut-off value of the second ultrafiltration membrane does not exceed 1000KD.
The disclosed invention is directed to methods of ultrafiltration; accordingly, the maximum cut-off value of the membrane for use in accordance with the invention (*i.e.,* in the second TFF unit operation) is the upper-limit size limit for ultrafiltration as defined in the art, *i.e.,* relating to the filtration of molecules at or below 1000 kD. Accordingly, this also limits the size and/or the molecular weight of molecules of interest, e.g., proteins, encompassed by the methods of the invention. Molecules of interest according to the present invention are at or below the upper size-limit for ultrafiltration, that is, at or below 1000 kD. In embodiments of the disclosed invention, the cut-off value of the filter membrane of the second TFF unit operation may be 1, 1.5, 2, 3, 4, 5, 10, 15, 20, 30, 40, 50, 60, 70, 80, 90 or 100 times the molecular weight of the molecule of interest, provided that the molecule of interest does pass through the filter membrane during the second TFF unit operation and provided that the membrane cut-off value is no more than 1000 kD. The molecule of interest according to the present invention may be a molecule having a molecular weight from about 10 to about 1000 kD. In certain embodiments of the invention, the molecule of interest is a protein having a molecular weight from about 25 kD to about 667 kD. In preferred embodiments, the molecule of interest is a protein having a molecular weight of about 25 kD to about 300 kD. In a most preferred embodiment, the molecule of interest is an antibody, antibody fragment and/or a protein having a molecular weight of 150 + 75 kD.

In specific embodiments, the cut-off value of the filter membrane of the second TFF unit operation as disclosed herein is at least twice the molecular weight of the molecule of interest, e.g., protein, but not greater than 1000 kD. In other words, in these embodiments, the cut-off value of the filter membrane of the second TFF unit operation as disclosed herein is 2 times the molecular weight of the molecule of interest or greater, and less than 1000 kD. In accordance with the this embodiment, the molecule of interest has a maximum molecular weight of about 500 kD.

In certain embodiments, the invention provides a method of separating a protein of interest from a cell culture solution containing the protein using dual stage TFF, wherein the dual stage TFF comprises at least
(i) a first tangential-flow ultrafiltration unit operation that filters a first product stream containing said protein through a first ultrafiltration membrane having a cut-off value less than the molecule weight of the protein of interest; and
(ii) a second tangential-flow ultrafiltration unit operation that filters a second product stream containing said protein through a second ultrafiltration membrane having a cut-off value greater than the molecular weight of the protein of interest.
The components (i) and (ii) of the present invention, may, in further embodiments be additionally characterized in that the protein is recovered in the retentate of the first TFF unit operation and is recovered in the permeate of the second TFF unit operation.

The invention also provides a method of separating a protein of interest from a cell culture solution containing the protein using dual stage TFF, wherein the dual stage TFF comprises at least
(i) a first tangential-flow ultrafiltration unit operation that filters a first product stream containing said protein through a first ultrafiltration membrane having a cut-off value 0.5 times the molecule weight of the protein of interest or less; and
(ii) a second tangential-flow ultrafiltration unit operation that filters a second product stream containing said protein through a second ultrafiltration membrane having a cut-off value of at least two times the molecular weight of the protein of interest but less than 1000 KD.
The components (i) and (ii) of the present invention, may, in further embodiments be additionally characterized in that the protein is recovered in the retentate of the first TFF unit operation and is recovered in the permeate of the second TFF unit operation.

As previously disclosed herein, in preferred embodiments of the invention, the feed stream of the dual stage TFF is a cell culture supernatant. The cell culture supernatant may be sterilized prior to implementation of the dual stage TFF methods/operations as disclosed herein and/or according to any method known in the art. For example, the present invention encompasses the filtration of the cell culture supernatant through a filter of between 0.1 µm and 0.45 µm upstream of the dual stage TFF methods/operations disclosed herein. Additionally, or alternately, the present invention encompasses methods and/or operations wherein the cell supernatant is filtered through a filter having a pore size of no greater than 0.22 µm, preferably a filter of 0.2 µm or 0.22 µm pore size, upstream of the remaining dual stage TFF operations as described herein. Accordingly, the cell culture supernatant may be processed by one or more additional operations prior to implementation of the dual stage TFF methods disclosed herein or may be fed directly into one of the at least two TFF unit operations of the invention. Therefore, in certain embodiments, the cell culture supernatant (which has optionally been filtered through a filter of between 0.1 and 0.45 µm pore size, preferably a filter of 0.2 µm or 0.22 µm pore size) is the first or second product stream filtered by the first or the second TFF unit operation as defined herein, respectively. Accordingly, in these embodiments, and with the exception of the optional sterilization, *e.g.*, by filtration as described herein, the first or second TFF unit operation as defined herein is the first unit operation to process the crude cell culture supernatant.

Therefore, the invention also provides a method of separating a protein of interest from a solution containing the protein using dual stage TFF, wherein the dual stage TFF comprises a first TFF unit operation and a second TFF unit operation, wherein
(i) the first TFF unit operation filters a first product stream containing the protein using a first ultrafiltration membrane having a cut-off value such that the protein of interest is recovered in the retentate of the first TFF unit operation, and/or wherein the cut-off value of the first ultrafiltration membrane is 0.5 times the molecular weight of the protein; and
(ii) the second TFF unit operation filters a second product stream containing the protein through a second ultrafiltration membrane having a cut-off value such that the protein of interest is recovered in the permeate of the second TFF unit operation, wherein the cut-off value of the second ultrafiltration membrane does not exceed 1000KD;
and wherein said solution is a cell culture supernatant that is filtered through a filter of between 0.1 and 0.45 µm pore size upstream of said dual stage TFF.

The dual stage TFF methods disclosed herein may also comprise one or more stages of diafiltration. In certain embodiments, the diafiltration may be combined with the first TFF unit operation as disclosed herein (*i.e.,* wherein the retentate of the first TFF unit operation is diafiltered, *e.g.*, for buffer exchange and/or for concentration of the molecule of interest as is known in the art). As used throughout the present disclosure, the terms "first" and "second" in connection with the specific at least two TFF unit operations defined herein are understood to merely be designators of the individual TFF unit operations and are not intended to imply any process or chronological order within the dual stage TFF method itself. Specifically, the first TFF unit operation as defined herein is characterized by comprising a filter membrane such that the molecule of interest does not pass through the membrane, and the second TFF unit operation as defined herein is characterized by comprising a filter membrane such that the molecule of interest does pass through the membrane. In a specific embodiment, the filter membrane used in the first TFF unit operation as disclosed herein is characterized by having a cut-off value that is about one-half (0.5) times the molecular weight of the molecule of interest or less, and the filter membrane used in the second TFF unit operation as disclosed herein is characterized by having a cut-off value that is at least 2 times the molecular weight of the molecule of interest but less than 1000 kD.

Because the identifiers first and second in connection with a TFF unit operation do not imply process or chronological order, the dual stage TFF methods disclosed herein encompass not only embodiments wherein the first TFF unit operation is upstream of the second TFF unit operation, but also embodiments wherein the first TFF unit operation is downstream of the second TFF unit operation. Where the first TFF unit operation is downstream of the second TFF unit operation, the feed stream of the first TFF unit operation may be the permeate stream from the second TFF unit operation. Where the feed stream of the first TFF unit operation is the permeate stream from the second TFF unit operation, the first and second TFF unit operations may be run in parallel (*e.g*., simultaneously) to eliminate the need for storage of the permeate from the second TFF unit operation.

In preferred embodiments, the first TFF unit operation is upstream of the second TFF unit operation. In this embodiment, the feed stream to the dual stage TFF may be a cell culture supernatant that is optionally filtered through an, at greatest, 0.22 µm filter, which feed stream is processed directly by the first TFF unit operation as disclosed herein *(i.e.,* forms the first feed stream absent further processing by one or more additional unit operations). In accordance with this embodiment, the first TFF unit operation is upstream of the second TFF unit operation, and the retentate stream of the first TFF unit operation may or may not directly form the feed stream of the second TFF unit operation *(i.e.,* the first TFF unit operation may be followed immediately downstream by the second TFF unit operation, or the first and second TFF unit operations may be separated by one or more additional unit operations). In preferred embodiments, the first TFF unit operation is followed immediately downstream by the second TFF unit operation.

The dual stage TFF methods disclosed herein may comprise one or more additional TFF unit operations, which one or more additional TFF unit operations may be upstream of the first TFF unit operation, upstream of the second TFF unit operation, upstream of the first and second TFF unit operations, downstream of the first TFF unit operation, downstream of the second TFF unit operation, downstream of the first and second TFF unit operations, and/or interspersed between the first and second TFF unit operations.

The one or more additional TFF unit operations of the dual stage TFF method may be a third TFF unit operation. The ultrafiltration membrane of the third TFF unit operation is selected such that the molecule of interest does not pass through the membrane during third TFF unit operation. Therefore, the criteria for selection of the filter membrane of the third TFF unit operation are the same as for the selection of the filter membrane of the first TFF unit operation. The filter membranes of the first and third TFF unit operation may be the same or different; the cut-off value or other permeability parameter (*e.g.*, pore size) of the membranes of the first and third TFF unit operation may be the same or different.

As described herein with respect to the first TFF unit operation, the cut-off value of the membrane of the third TFF unit operation will typically be less than the molecular weight of the molecule of interest. However, based on membrane performance, membranes with cut-off values greater than the molecular weight of the molecule of interest may be used in the third TFF unit operation, provided that the molecule of interest does not pass through the membrane during the third TFF unit operation. Accordingly, the particular membrane selected for use in the third TFF unit operation is not in any other manner limited and may be selected based on manufacturing ease or commercial availability provided it meets the other criteria set forth herein. In embodiments of the disclosed invention, the cut-off value of the filter membrane of the third TFF unit operation may be 1.5, 1, ½, 1/3, 1/5, 1/10 or 1/15 times the molecular weight of the molecule of interest, *e.g*., protein, provided that the molecule of interest does not pass through the membrane during the third TFF unit operation . In other embodiments, the cut-off value of the filter membrane of the third TFF unit operation is less than 1/15 of the molecular weight of the molecule of interest. As with the filter membrane of the first TFF unit operation, selection of the membrane for the third TFF unit operation also may depend on additional parameters such as availability (*e.g*., whether commercially available or whether available by self-manufacture) or performance (*e.g.*, performance in separating the molecule of interest from a specific known contaminant/impurity). In specific embodiments, the cut-off value of the filter membrane of the third TFF unit operation as disclosed herein is no greater than about one-half of the molecular weight of the molecule of interest, *e.g*., protein. In other words, in these embodiments, the cut-off value of the filter membrane of the third TFF unit operation as disclosed herein is 0.5 times the molecular weight of the molecule of interest or less.
The third TFF unit operation may have an ultrafiltration membrane that is the same or a different material from the ultrafiltration membrane of the first TFF unit operation. Accordingly, the ultrafiltration membrane of the third TFF unit operation may have a cut-off value that is the same or different from the cut-off value of the ultrafiltration membrane of the first TFF unit operation. The third TFF unit operation may be combined with diafiltration as described herein or according to any method known in the art. In a specific embodiment, the cut-off value of the filter membrane of the third unit operation is the same as the cut-off value of the first TFF unit operation.
In certain embodiments, the dual-stage TFF method comprises a third TFF unit operation that is downstream of both the first and second TFF unit operations, wherein the first TFF unit operation is upstream of the second TFF unit operation. In this embodiment, the third TFF unit operation may be directly downstream or run in parallel with the second TFF unit operation, *i.e.,* so that the permeate of the second TFF unit operation (containing the molecule of interest) forms the product stream for the third TFF unit operation. The third TFF unit operation may also be separated by one or more additional unit operations or processes from the second TFF unit operation. In certain embodiments, the third TFF unit operation has a filter membrane having a cut-off value that is 0.5 times the molecular weight of the molecule of interest or less. In a further specific aspect, the third TFF unit operation has a filter membrane having a cut-off value that is the same as the cut-off value of the filter membrane of the first TFF unit operation. In all embodiments comprising the third TFF unit operation, the feed stream of the dual stage TFF method may be a cell culture supernatant that has been optionally filtered through an at least 0.22 µm filter, which feed stream is processed directly by the first TFF unit operation as disclosed herein (*i.e.,* forms the first feed or product stream absent further processing by one or more additional unit operations). In accordance with this embodiment, the first TFF unit operation is upstream of the second TFF unit operation, and the retentate stream of the first TFF unit operation may or may not directly form the product/feed stream of the second TFF unit operation (*i.e.,* the first TFF unit operation may be followed immediately downstream by the second TFF unit operation, or one or more additional unit operations may separate the first and second TFF unit operations).
In certain embodiments, the molecule of interest is a biomolecule that is a protein having a molecular weight of about 10 to about 300 kD, about 25 to about 300 kD, about 50 to about 300 kD, about 75 to about 300 kD, about 100 to about 300 kD, about 120 to about 300 kD, about 135 to about 300 kD, about 10 to about 200 kD, about 25 to about 200 kD, about 50 to about 200 kD, about 75 to about 200 kD, about 100 to about 200 kD, about 120 to about 200 kD, about 135 to about 200 kD, about 10 to about 175 kD, about 25 to about 175 kD, about 50 to about 175 kD, about 75 to about 175 kD, about 120 to about 175 kD or about 135 to about 175 kD.
In preferred embodiments, the molecule of interest is a biomolecule that is a protein having a molecular weight of 150 + 75 kD. Accordingly, in embodiments, the protein of interest may be about 75 kD, about 80 kD, about 90 kD, about 95 kD, about 100 kD, about 105 kD, about 110 kD, about 115 kD, about 120 kD, about 125 kD, about 130 kD, about 135 kD, about 140 kD, about 150 kD, about 155 kD, about 160 kD, about 165 kD, about 170 kD, about 175 kD, about 180 kD, about 185 kD, about 190 kD, about 195 kD, about 200 kD, about 205 kD, about 210 kD, about 215 kD, about 220 kD or about 215 kD. Alternately or additionally, the protein of interest may be characterized by having a molecular weight that is 150 ± 15 kD, 150 ± 30 kD, 150 ± 50 kD. Examples of proteins having a molecular weight of 150 ± 75 kD include immunoglobulins and antibodies. Examples of dual stage TFF methods that may be used with proteins having a molecular weight of 150 ± 75 kD (*e.g.,* 150 ± 30 and/or 150 ± 15 kD), comprise, for the first TFF unit operation, a filter membrane having a cut-off value of 50 kD or less; for the second TFF unit operation, a filter membrane having a cut-off value of at least 300 kD but not greater than 1000 KD; and an optional third TFF unit operation having a cut-off value of 50 kD or less. A specific example of a dual stage TFF method for such proteins comprises the use of, for the first TFF unit operation, a filter membrane having a cut-off value of 30 kD to 50 kD (preferably 50 kD); for the second TFF unit operation, a filter membrane having a cut-off value of 300 kD; and an optional third TFF unit operation having a filter membrane with a cut-off value of 30 kD to 50 kD (preferably 50 kD).

As disclosed herein, the invention encompasses methods of separating an immunoglobulin (*e.g*., antibody) of interest from a cell culture supernatant (*e.g.*, supernatant of a hybridoma cell culture expressing the antibody of interest) using dual stage TFF, wherein the dual stage TFF comprises a first TFF unit operation, a second TFF unit operation, and an optional third TFF unit operation, and wherein
(i) said first TFF unit operation filters a first product stream containing the immunoglobulin of interest using a first ultrafiltration membrane having a cut-off value of 30 kD to 50 kD (preferably 50 kD);
(ii) said second TFF unit operation filters a second product stream containing the immunoglobulin of interest using a second ultrafiltration membrane having a cut-off value that is at least 300 kD but not more than 1000 kD (preferably 300 kD); and
(iii) said optional third TFF unit operation filters a third product stream containing the immunoglobulin of interest using a third ultrafiltration membrane having a cut-off value of 30 kD to 50 kD or less (preferably 50 kD).
In a preferred aspect associated with the embodiment described in this paragraph, the first product steam is a cell culture supernatant that has optionally been sterilized. The optional sterilization may be effected by any method known in the art and/or described herein. For example, sterilization of the first product stream upstream of the first TFF unit operation may be effected by filtration through a filter having a pore size of between 0.1 µm and 0.45 µm, and is preferably effected by filtration through a filter having a pore size of 0.2 µm or 0.22 µm. In further preferred aspect associated with the embodiment described in this paragraph, the second TFF unit operation is operated directly downstream of the first TFF unit operation; that is, in this aspect, there are no intervening unit operations between the first and second TFF unit operations. In further preferred aspect associated with the embodiment described in this paragraph, the optional third TFF unit operation is, if present, directly downstream or run in parallel with the second TFF unit operation; that is, in this aspect, there are no intervening unit operations between the second and third (where present) TFF unit operations.

The preferred aspects and/or embodiments outlined throughout the disclosure are expressly disclosed as separate aspects, which may be individually implemented into the dual stage TFF method, and are also disclosed as combinatorial aspects that may be combined with one or more other preferred aspect. Thus, for example, the invention encompasses a method of separating an immunoglobulin (*e.g.,* antibody) of interest from a cell culture supernatant (*e.g.,* supernatant of a hybridoma cell culture expressing the antibody of interest) using dual stage TFF, wherein the dual stage TFF comprises a first TFF unit operation, a second TFF unit operation, and an optional third TFF unit operation, and wherein
(i) said first TFF unit operation filters a first product stream containing the immunoglobulin of interest using a first ultrafiltration membrane having a cut-off value of 50 kD;
(ii) said second TFF unit operation filters a second product stream containing the immunoglobulin of interest using a second ultrafiltration membrane having a cut-off value of 300 kD; and
(iii) said optional third TFF unit operation filters a third product stream containing the immunoglobulin of interest using a third ultrafiltration membrane having a cut-off value of 50 kD.
wherein said first product stream is a cell culture supernatant that has optionally been sterilized by filtration through a filter membrane having a pore size of no greater than 0.22 µm; wherein said second TFF unit operation is immediately downstream of said first TFF unit operation; and wherein said optional third TFF unit operation is, if present, immediately downstream or run in parallel with said second TFF unit operation.

In a specific embodiment of the dual stage TFF method described immediately above, the first product stream is the cell culture supernatant that has only optionally been filtered with a 0.22 µm filter, and the first TFF unit operation is immediately upstream of the second TFF unit operation, which second TFF unit operation is immediately upstream of the optional third TFF unit operations (*i.e*., this specific embodiment of the dual stage TFF method does not comprise the use of other unit operations between the first, second, and optional third TFF unit operations). Further, the third TFF unit operation may be run in parallel with the second TFF unit operation or may be implemented after completion of the second TFF unit operation (*i.e.,* each TFF unit operation is run in batch). In other related embodiments, the dual stage TFF method comprises the use of one or more additional unit operations that are interspersed between the first and second, and/or between the second and optional third TFF unit operations.

As used herein, the phrases, "operated immediately upstream", "operated directly downstream", "is immediately upstream", "is immediately downstream" and analogous phrases do not imply a chronological component; thus, *e.g.,* the operation of the first and second TFF unit operation or the second and third TFF unit operation need not both occur or proceed within any specific time period. Rather the phrases indicate that the product stream is processed by no other unit operations between the first and second or between the second and third unit operations; thus, for example, the first TFF unit operation can be run in, *e.g.,* batch mode, the product stream, *i.e.,* retentate stream, stored, and/or transported and stored, and then at some future time, the product stream is processed by the second TFF unit operation (so long as no intervening unit operation occurred).

In certain aspects, the invention encompasses methods of separating an immunoglobulin (*e.g*., antibody) of interest from a cell culture supernatant comprising the immunoglobulin using dual stage TFF, wherein the dual stage TFF comprises a first TFF unit operation and a second TFF unit operation, and wherein
(i) said first TFF unit operation filters a first product stream containing the immunoglobulin of interest using a first ultrafiltration membrane having a cut-off value of 50 kD or less, preferably 50 kD; and
(ii) said second TFF unit operation filters a second product stream containing the immunoglobulin of interest using a second ultrafiltration membrane having a cut-off value between 300 kD and 1000 kD, preferably 300 kD.

In other aspects, the invention encompasses methods of separating an immunoglobulin (*e.g*., antibody) of interest from a cell culture supernatant comprising the immunoglobulin using dual stage TFF, wherein the dual stage TFF comprises a first TFF unit operation and a second TFF unit operation,
wherein
(i) said first TFF unit operation filters a first product stream containing the immunoglobulin of interest using a first ultrafiltration membrane having a cut-off value of 50 kD or less, preferably 50 kD; and
(ii) said second TFF unit operation filters a second product stream containing the immunoglobulin of interest using a second ultrafiltration membrane having a cut-off value of between 300 kD and 1000 kD, preferably 300 kD;
and wherein
the second TFF unit operation is operated directly downstream of the first TFF unit operation; that is, in this aspect, there are no intervening unit operations between the first and second TFF unit operations.

In other aspects, the invention encompasses methods of separating an immunoglobulin (*e.g.*, antibody) of interest from a cell culture supernatant comprising the immunoglobulin using dual stage TFF, wherein the dual stage TFF comprises a first TFF unit operation and a second TFF unit operation,
wherein
(i) said first TFF unit operation filters a first product stream containing the immunoglobulin of interest using a first ultrafiltration membrane having a cut-off value of 50 kD or less, preferably 50 kD; and
(ii) said second TFF unit operation filters a second product stream containing the immunoglobulin of interest using a second ultrafiltration membrane having a cut-off value of between 300 kD and 1000 kD, preferably 300 kD.
and wherein
the first product steam is a cell culture supernatant that has been sterilized by filtration through a filter having a pore size of between 0.1 µm and 0.45 µm (preferably 0.2 µm or 0.22 µm).

In other aspects, the invention encompasses methods of separating an immunoglobulin (*e.g.*, antibody) of interest from a cell culture supernatant comprising the immunoglobulin using dual stage TFF, wherein the dual stage TFF comprises a first TFF unit operation and a second TFF unit operation,
wherein
(i) said first TFF unit operation filters a first product stream containing the immunoglobulin of interest using a first ultrafiltration membrane having a cut-off value of 50 kD or less, preferably 50 kD; and
(ii) said second TFF unit operation filters a second product stream containing the immunoglobulin of interest using a second ultrafiltration membrane having a cut-off value of between 300 kD and 1000 kD, preferably 300 kD.
and wherein
said methods further comprise a chromatography process (*e.g*., an ion exchange chromatography process and/or an affinity chromatography process) downstream of said first and said second TFF unit operations.

In other aspects, the invention encompasses methods of separating an immunoglobulin (*e.g.*, antibody) of interest from a cell culture supernatant comprising the immunoglobulin using dual stage TFF, wherein the dual stage TFF comprises a first TFF unit operation and a second TFF unit operation,
wherein
(i) said first TFF unit operation filters a first product stream containing the immunoglobulin of interest using a first ultrafiltration membrane having a cut-off value of 50 kD or less, preferably 50 kD; and
(ii) said second TFF unit operation filters a second product stream containing the immunoglobulin of interest using a second ultrafiltration membrane having a cut-off value of between 300 kD and 1000 kD, preferably 300 kD;
wherein
the first product steam is a cell culture supernatant that has been sterilized by filtration through a filter having a pore size of between 0.1 µm and 0.45 µm (preferably 0.2 µm or 0.22 µm);
and wherein
the second TFF unit operation is operated directly downstream of the first TFF unit operation (*i.e*., in this aspect, there are no intervening unit operations between the first and second TFF unit operations).

In other aspects, the invention encompasses methods of separating an immunoglobulin (*e.g.*, antibody) of interest from a cell culture supernatant comprising the immunoglobulin using dual stage TFF, wherein the dual stage TFF comprises a first TFF unit operation and a second TFF unit operation,
wherein
(i) said first TFF unit operation filters a first product stream containing the immunoglobulin of interest using a first ultrafiltration membrane having a cut-off value of 50 kD or less, preferably 50 kD; and
(ii) said second TFF unit operation filters a second product stream containing the immunoglobulin of interest using a second ultrafiltration membrane having a cut-off value of between 300 kD and 1000 kD, preferably 300 kD;
wherein
the first product steam is a cell culture supernatant that has been sterilized by filtration through a filter having a pore size of between 0.1 µm and 0.45 µm (preferably 0.2 µm or 0.22 µm);
and wherein
said methods further comprise a chromatography process (*e.g.*, an ion exchange chromatography process and/or an affinity chromatography process) downstream of said first and said second TFF unit operations.

In other aspects, the invention encompasses methods of separating an immunoglobulin (*e.g.*, antibody) of interest from a cell culture supernatant comprising the immunoglobulin using dual stage TFF, wherein the dual stage TFF comprises a first TFF unit operation and a second TFF unit operation,
wherein
(i) said first TFF unit operation filters a first product stream containing the immunoglobulin of interest using a first ultrafiltration membrane having a cut-off value of 50 kD or less, preferably 50 kD; and
(ii) said second TFF unit operation filters a second product stream containing the immunoglobulin of interest using a second ultrafiltration membrane having a cut-off value of between 300 kD and 1000 kD, preferably 300 kD;
wherein
the second TFF unit operation is operated directly downstream of the first TFF unit operation (*i.e.,* in this aspect, there are no intervening unit operations between the first and second TFF unit operations);
and wherein
said methods further comprise a chromatography process (*e.g.*, an ion exchange chromatography process and/or an affinity chromatography process) downstream of said first and said second TFF unit operations.

In other aspects, the invention encompasses methods of separating an immunoglobulin (*e.g*., antibody) of interest from a cell culture supernatant comprising the immunoglobulin using dual stage TFF, wherein the dual stage TFF comprises a first TFF unit operation and a second TFF unit operation,
wherein
(i) said first TFF unit operation filters a first product stream containing the immunoglobulin of interest using a first ultrafiltration membrane having a cut-off value of 50 kD or less, preferably 50 kD; and
(ii) said second TFF unit operation filters a second product stream containing the immunoglobulin of interest using a second ultrafiltration membrane having a cut-off value of between 300 kD and 1000 kD, preferably 300 kD;
wherein
the first product steam is a cell culture supernatant that has been sterilized by filtration through a filter having a pore size of between 0.1 µm and 0.45 µm (preferably 0.2 µm or 0.22 µm);
wherein
the second TFF unit operation is operated directly downstream of the first TFF unit operation (*i.e.,* in this aspect, there are no intervening unit operations between the first and second TFF unit operations);
and wherein
said methods further comprise a chromatography process (*e.g.,* an ion exchange chromatography process and/or an affinity chromatography process) downstream of said first and said second TFF unit operations.

In certain aspects, the invention encompasses methods of separating an immunoglobulin (*e.g.*, antibody) of interest from a cell culture supernatant comprising the immunoglobulin using dual stage TFF, wherein the dual stage TFF comprises a first TFF unit operation, a second TFF unit operation and a third TFF unit operation,
wherein
(i) said first TFF unit operation filters a first product stream containing the immunoglobulin of interest using a first ultrafiltration membrane having a cut-off value of 50 kD or less, preferably 50 kD;
(ii) said second TFF unit operation filters a second product stream containing the immunoglobulin of interest using a second ultrafiltration membrane having a cut-off value of between 300 kD and 1000 kD, preferably 300 kD; and
(iii) said third TFF unit operation filters a third product stream containing the immunoglobulin of interest using a third ultrafiltration membrane having a cut-off value of 50 kD or less, preferably 50 kD.

In other aspects, the invention encompasses methods of separating an immunoglobulin (*e.g*., antibody) of interest from a cell culture supernatant comprising the immunoglobulin using dual stage TFF, wherein the dual stage TFF comprises a first TFF unit operation, a second TFF unit operation and a third TFF unit operation,
wherein
(i) said first TFF unit operation filters a first product stream containing the immunoglobulin of interest using a first ultrafiltration membrane having a cut-off value of 50 kD or less, preferably 50 kD;
(ii) said second TFF unit operation filters a second product stream containing the immunoglobulin of interest using a second ultrafiltration membrane having a cut-off value of of between 300 kD and 1000 kD, preferably 300 kD; and
(iii) said third TFF unit operation filters a third product stream containing the immunoglobulin of interest using a third ultrafiltration membrane having a cut-off value of 50 kD or less, preferably 50 kD;
wherein
the second TFF unit operation is operated directly downstream of the first TFF unit operation (*i.e.,* in this aspect, there are no intervening unit operations between the first and second TFF unit operations);
and wherein
the third TFF unit operation is operated directly downstream or in parallel with the second TFF unit operation (*i.e.,* in this aspect, there are no intervening unit operations between the second and third TFF unit operations).

In other aspects, the invention encompasses methods of separating an immunoglobulin (*e.g.*, antibody) of interest from a cell culture supernatant comprising the immunoglobulin using dual stage TFF, wherein the dual stage TFF comprises a first TFF unit operation, a second TFF unit operation and a third TFF unit operation,
wherein
(i) said first TFF unit operation filters a first product stream containing the immunoglobulin of interest using a first ultrafiltration membrane having a cut-off value of 50 kD or less, preferably 50 kD;
(ii) said second TFF unit operation filters a second product stream containing the immunoglobulin of interest using a second ultrafiltration membrane having a cut-off value of of between 300 kD and 1000 kD, preferably 300 kD; and
(iii) said third TFF unit operation filters a third product stream containing the immunoglobulin of interest using a third ultrafiltration membrane having a cut-off value of 50 kD or less, preferably 50 kD;
and wherein
the first product steam is a cell culture supernatant that has been sterilized by filtration through a filter having a pore size of between 0.1 µm and 0.45 µm (preferably 0.2 µm or 0.22 µm).

In other aspects, the invention encompasses methods of separating an immunoglobulin (*e.g*., antibody) of interest from a cell culture supernatant comprising the immunoglobulin using dual stage TFF, wherein the dual stage TFF comprises a first TFF unit operation, a second TFF unit operation and a third TFF unit operation,
wherein
(i) said first TFF unit operation filters a first product stream containing the immunoglobulin of interest using a first ultrafiltration membrane having a cut-off value of 50 kD or less, preferably 50 kD;
(ii) said second TFF unit operation filters a second product stream containing the immunoglobulin of interest using a second ultrafiltration membrane having a cut-off value of of between 300 kD and 1000 kD, preferably 300 kD; and
(iii) said third TFF unit operation filters a third product stream containing the immunoglobulin of interest using a third ultrafiltration membrane having a cut-off value of 50 kD or less, preferably 50 kD;
and wherein
said methods further comprise a chromatography process (*e.g.,* an ion exchange chromatography process and/or an affinity chromatography process) downstream of said first, said second and said third TFF unit operations.

In other aspects, the invention encompasses methods of separating an immunoglobulin (*e.g*., antibody) of interest from a cell culture supernatant comprising the immunoglobulin using dual stage TFF, wherein the dual stage TFF comprises a first TFF unit operation, a second TFF unit operation and a third TFF unit operation,
wherein
(i) said first TFF unit operation filters a first product stream containing the immunoglobulin of interest using a first ultrafiltration membrane having a cut-off value of 50 kD or less, preferably 50 kD;
(ii) said second TFF unit operation filters a second product stream containing the immunoglobulin of interest using a second ultrafiltration membrane having a cut-off value of of between 300 kD and 1000 kD, preferably 300 kD; and
(iii) said third TFF unit operation filters a third product stream containing the immunoglobulin of interest using a third ultrafiltration membrane having a cut-off value of 50 kD or less, preferably 50 kD;
wherein
the second TFF unit operation is operated directly downstream of the first TFF unit operation (*i.e.,* in this aspect, there are no intervening unit operations between the first and second TFF unit operations);
wherein
the third TFF unit operation is operated directly downstream or in parallel with the second TFF unit operation *(i.e.,* in this aspect, there are no intervening unit operations between the second and third TFF unit operations);
and wherein
the first product steam is a cell culture supernatant that has been sterilized by filtration through a filter having a pore size of between 0.1 µm and 0.45 µm (preferably 0.2 µm or 0.22 µm).

In other aspects, the invention encompasses methods of separating an immunoglobulin (*e.g*., antibody) of interest from a cell culture supernatant comprising the immunoglobulin using dual stage TFF, wherein the dual stage TFF comprises a first TFF unit operation, a second TFF unit operation and a third TFF unit operation,
wherein
(i) said first TFF unit operation filters a first product stream containing the immunoglobulin of interest using a first ultrafiltration membrane having a cut-off value of 50 kD or less, preferably 50 kD;
(ii) said second TFF unit operation filters a second product stream containing the immunoglobulin of interest using a second ultrafiltration membrane having a cut-off value of of between 300 kD and 1000 kD, preferably 300 kD; and
(iii) said third TFF unit operation filters a third product stream containing the immunoglobulin of interest using a third ultrafiltration membrane having a cut-off value of 50 kD or less, preferably 50 kD;
wherein
the second TFF unit operation is operated directly downstream of the first TFF unit operation (*i.e.,* in this aspect, there are no intervening unit operations between the first and second TFF unit operations);
wherein
the third TFF unit operation is operated directly downstream or in parallel with the second TFF unit operation (*i.e.,* in this aspect, there are no intervening unit operations between the second and third TFF unit operations);
and wherein
said methods further comprise a chromatography process (*e.g*., an ion exchange chromatography process and/or an affinity chromatography process) downstream of said first, said second and said third TFF unit operations.

In other aspects, the invention encompasses methods of separating an immunoglobulin (*e.g*., antibody) of interest from a cell culture supernatant comprising the immunoglobulin using dual stage TFF, wherein the dual stage TFF comprises a first TFF unit operation, a second TFF unit operation and a third TFF unit operation,
wherein
(i) said first TFF unit operation filters a first product stream containing the immunoglobulin of interest using a first ultrafiltration membrane having a cut-off value of 50 kD or less, preferably 50 kD;
(ii) said second TFF unit operation filters a second product stream containing the immunoglobulin of interest using a second ultrafiltration membrane having a cut-off value of of between 300 kD and 1000 kD, preferably 300 kD; and
(iii) said third TFF unit operation filters a third product stream containing the immunoglobulin of interest using a third ultrafiltration membrane having a cut-off value of 50 kD or less, preferably 50 kD;
wherein
the first product steam is a cell culture supernatant that has been sterilized by filtration through a filter having a pore size of between 0.1 µm and 0.45 µm (preferably 0.2 µm or 0.22 µm);
and wherein
said methods further comprise a chromatography process (*e.g.*, an ion exchange chromatography process and/or an affinity chromatography process) downstream of said first, said second and said third TFF unit operations.

In other aspects, the invention encompasses methods of separating an immunoglobulin (*e.g.*, antibody) of interest from a cell culture supernatant comprising the immunoglobulin using dual stage TFF, wherein the dual stage TFF comprises a first TFF unit operation, a second TFF unit operation and a third TFF unit operation,
wherein
(i) said first TFF unit operation filters a first product stream containing the immunoglobulin of interest using a first ultrafiltration membrane having a cut-off value of 50 kD or less, preferably 50 kD;
(ii) said second TFF unit operation filters a second product stream containing the immunoglobulin of interest using a second ultrafiltration membrane having a cut-off value of of between 300 kD and 1000 kD, preferably 300 kD; and
(iii) said third TFF unit operation filters a third product stream containing the immunoglobulin of interest using a third ultrafiltration membrane having a cut-off value of 50 kD or less, preferably 50 kD;
wherein
the second TFF unit operation is operated directly downstream of the first TFF unit operation (*i.e.,* in this aspect, there are no intervening unit operations between the first and second TFF unit operations);
wherein
the third TFF unit operation is operated directly downstream or in parallel with the second TFF unit operation *(i.e.,* in this aspect, there are no intervening unit operations between the second and third TFF unit operations);
wherein
the first product steam is a cell culture supernatant that has been sterilized by filtration through a filter having a pore size of between 0.1 µm and 0.45 µm (preferably 0.2 µm or 0.22 µm);
and wherein
said methods further comprise a chromatography process (*e.g.*, an ion exchange chromatography process and/or an affinity chromatography process) downstream of said first, said second and said third TFF unit operations.

The dual stage TFF methods disclosed herein are effective at reducing the levels of contaminants and impurities from the solution containing the molecule of interest. In particular, the dual stage TFF methods of the invention are effective at reducing contaminant and/or impurity levels in a crude feed stream of a cell culture solution (*e.g.*, a cell culture supernatant optionally filtered through a 0.22 µm membrane) prior to downstream processing in one or more unit operations (*e.g*., chromatographic columns). As used herein, the terms "contaminant", "impurity" and analogous terms have their standard meaning known in the art and, in particular, indicate undesired components in the solution containing the molecule of interest. Examples of such undesired components that may be found in the crude feed stream of a cell culture solution (*e.g.,* cell culture supernatant optionally filtered through a 0.22 µm membrane) include undesired proteins, undesired small molecules, fragments of the molecule of interest, aggregates of the molecule of interest, undesired components of the cell culture medium, undesired component produced by the cell (whether endogenous or heterologous). Generally, such contaminants and/or impurities are referenced in the literature as host-cell proteins ("HCP") and host-cell DNA ("HCDNA").

In examples, the cell culture solution (*e.g.*, cell culture supernatant) that forms the feed stream of the dual stage TFF of the present invention may have a HCDNA concentration of at least 500,000 pg/mg of protein of interest, at least at least 750,000 pg/mg of protein of interest, or at least 1,000,000 pg/mg of protein of interest prior to operation of the dual stage TFF methods as disclosed herein. Alternately or additionally, the cell culture solution (*e.g.*, cell culture supernatant) that forms the feed stream of the dual stage TFF of the present invention may have a HCP concentration of at least 100,000 ng/mg of protein of interest, at least 150,000 ng/mg of protein of interest, or at least 200,000 ng/mg of protein of interest prior to operation of the dual stage TFF methods as disclosed herein.
The dual stage TFF methods of the present invention may reduce the level of HCP in the cell culture solution containing the protein of interest (*e.g.,* cell culture supernatant) by at least 10%, at least 15%, at least 20%, at least 25%, at least 30%, at least 35%, at least 40%, at least 45% or at least 50%. Alternately or additionally, the dual stage TFF methods of the present invention may reduce the level of HCDNA in the cell culture solution containing the protein of interest (*e.g.,* cell culture supernatant) by at least 50%, at least 55%, at least 60%, at least 65%, at least 70%, at least 75%, at least 80%, at least 85%, at least 90% or at least 95%. In certain embodiments, the dual stage TFF method disclosed herein reduces the level of HCDNA in the solution containing the protein of interest by 85 to 95%. The percentage reduction may be determined according to any method disclosed herein and/or known in the art suitable for the determination of concentration of HCP and/or HCDNA, and is calculated by comparing the level of HCP and/or HCDNA in the solution containing the protein of interest subsequent to the implementation of dual-stage TFF with that level in the solution containing the protein of interest prior to implementation of dual stage TFF (*e.g.,* in the crude feed stream). Because dual stage TFF may comprise one or more steps of diafiltration and/or buffer exchange as disclosed herein, the composition of the solution containing the protein of interest with respect to components other than HCP and HCDNA can also significantly change during the method.

In a specific embodiment, wherein the solution containing the protein of interest prior to implementation of the dual stage TFF method is a cell culture supernatant, the dual stage TFF method reduces the concentration of HCDNA in the solution to 600,000 pg per mg of said protein or less, to 500,000 pg per mg of said protein or less, to 400,000 pg per mg of said protein or less, to 300,000 pg per mg of said protein or less or to 200,000 pg per mg of said protein or less, and/or reduces the concentration of HCP in the solution to 450,000 ng per mg of said protein or less, to 400,000 ng per mg of said protein or less, or to 350,000 ng per mg of said protein or less.

The methods of the invention are, in certain embodiments, suitable for the purification of a protein of interest from a fermentation reaction wherein the fermentation reaction produces a high concentration of protein. Thus, in certain embodiments, the methods of the invention encompass the purification of a protein of interest form a cell culture solution wherein the protein has a concentration at least 200 mg/l, 400 mg/l, 600 mg/l, 700 mg/l, 800 mg/l, 900 mg/l, 1 g/l, 1.5 g/l, 2 g/l, 2.5 g/l, 3 g/l, 3.5 g/l, 4 g/l, 4.5 g/l or 5 g/l in the solution.

In certain aspects, the invention encompasses methods of separating an immunoglobulin (*e.g*., antibody) of interest from a cell culture supernatant comprising the immunoglobulin using dual stage TFF, wherein the dual stage TFF comprises a first TFF unit operation and a second TFF unit operation,
wherein
(i) said first TFF unit operation filters a first product stream containing the immunoglobulin of interest using a first ultrafiltration membrane having a cut-off value of 50 kD or less, preferably 50 kD; and
(ii) said second TFF unit operation filters a second product stream containing the immunoglobulin of interest using a second ultrafiltration membrane having a cut-off value between 300 kD and 1000 kD, preferably 300 kD;
and wherein, following operation of the first and second TFF unit operations,
(a) the concentration of HCP in the solution containing the immunoglobulin is reduced by at least 10% relative to the HCP concentration in the cell culture supernatant; and/or
(b) the concentration of HCP in the solution containing the immunoglobulin is 400,000 ng per mg of the immunoglobulin or less; and/or
(c) the concentration of HCDNA in the solution containing the immunoglobulin is reduced by at least 30% relative to the HCDNA concentration in the cell culture supernatant; and/or
(d) the concentration of HCDNA in the solution containing the immunoglobulin is 1,500,000 pg per mg of immunoglobulin or less.

In other aspects, the invention encompasses methods of separating an immunoglobulin (*e.g*., antibody) of interest from a cell culture supernatant comprising the immunoglobulin using dual stage TFF, wherein the dual stage TFF comprises a first TFF unit operation and a second TFF unit operation,
wherein
(i) said first TFF unit operation filters a first product stream containing the immunoglobulin of interest using a first ultrafiltration membrane having a cut-off value of 50 kD or less, preferably 50 kD; and
(ii) said second TFF unit operation filters a second product stream containing the immunoglobulin of interest using a second ultrafiltration membrane having a cut-off value of between 300 kD and 1000 kD, preferably 300 kD;
wherein
the second TFF unit operation is operated directly downstream of the first TFF unit operation (*i.e.,* in this aspect, there are no intervening unit operations between the first and second TFF unit operations);
and wherein, following operation of the first and second TFF unit operations,
(a) the concentration of HCP in the solution containing the immunoglobulin is reduced by at least 10% relative to the HCP concentration in the cell culture supernatant; and/or
(b) the concentration of HCP in the solution containing the immunoglobulin is 400,000 ng per mg of the immunoglobulin or less; and/or
(c) the concentration of HCDNA in the solution containing the immunoglobulin is reduced by at least 30% relative to the HCDNA concentration in the cell culture supernatant; and/or
(d) the concentration of HCDNA in the solution containing the immunoglobulin is 1,500,000 pg per mg of immunoglobulin or less.

In other aspects, the invention encompasses methods of separating an immunoglobulin (*e.g.*, antibody) of interest from a cell culture supernatant comprising the immunoglobulin using dual stage TFF, wherein the dual stage TFF comprises a first TFF unit operation and a second TFF unit operation,
wherein
(i) said first TFF unit operation filters a first product stream containing the immunoglobulin of interest using a first ultrafiltration membrane having a cut-off value of 50 kD or less, preferably 50 kD; and
(ii) said second TFF unit operation filters a second product stream containing the immunoglobulin of interest using a second ultrafiltration membrane having a cut-off value of between 300 kD and 1000 kD, preferably 300 kD.
wherein
the first product steam is a cell culture supernatant that has been sterilized by filtration through a filter having a pore size of between 0.1 µm and 0.45 µm (preferably 0.2 µm or 0.22 µm);
and wherein, following operation of the first and second TFF unit operations,
(a) the concentration of HCP in the solution containing the immunoglobulin is reduced by at least 10% relative to the HCP concentration in the cell culture supernatant; and/or
(b) the concentration of HCP in the solution containing the immunoglobulin is 400,000 ng per mg of the immunoglobulin or less; and/or
(c) the concentration of HCDNA in the solution containing the immunoglobulin is reduced by at least 30% relative to the HCDNA concentration in the cell culture supernatant; and/or
(d) the concentration of HCDNA in the solution containing the immunoglobulin is 1,500,000 pg per mg of immunoglobulin or less.

In other aspects, the invention encompasses methods of separating an immunoglobulin (e.g., antibody) of interest from a cell culture supernatant comprising the immunoglobulin using dual stage TFF, wherein the dual stage TFF comprises a first TFF unit operation and a second TFF unit operation,
wherein
(i) said first TFF unit operation filters a first product stream containing the immunoglobulin of interest using a first ultrafiltration membrane having a cut-off value of 50 kD or less, preferably 50 kD; and
(ii) said second TFF unit operation filters a second product stream containing the immunoglobulin of interest using a second ultrafiltration membrane having a cut-off value of between 300 kD and 1000 kD, preferably 300 kD.
wherein, following operation of the first and second TFF unit operations,
(a) the concentration of HCP in the solution containing the immunoglobulin is reduced by at least 10% relative to the HCP concentration in the cell culture supernatant; and/or
(b) the concentration of HCP in the solution containing the immunoglobulin is 400,000 ng per mg of the immunoglobulin or less; and/or
(c) the concentration of HCDNA in the solution containing the immunoglobulin is reduced by at least 30% relative to the HCDNA concentration in the cell culture supernatant; and/or
(d) the concentration of HCDNA in the solution containing the immunoglobulin is 1,500,000 pg per mg of immunoglobulin or less;
and wherein
said methods further comprise a chromatography process (*e.g.,* an ion exchange chromatography process and/or an affinity chromatography process) downstream of said first and said second TFF unit operations.

In other aspects, the invention encompasses methods of separating an immunoglobulin (*e.g.*, antibody) of interest from a cell culture supernatant comprising the immunoglobulin using dual stage TFF, wherein the dual stage TFF comprises a first TFF unit operation and a second TFF unit operation,
wherein
(i) said first TFF unit operation filters a first product stream containing the immunoglobulin of interest using a first ultrafiltration membrane having a cut-off value of 50 kD or less, preferably 50 kD; and
(ii) said second TFF unit operation filters a second product stream containing the immunoglobulin of interest using a second ultrafiltration membrane having a cut-off value of between 300 kD and 1000 kD, preferably 300 kD;
wherein
the first product steam is a cell culture supernatant that has been sterilized by filtration through a filter having a pore size of between 0.1 µm and 0.45 µm (preferably 0.2 µm or 0.22 µm);
wherein
the second TFF unit operation is operated directly downstream of the first TFF unit operation *(i.e.,* in this aspect, there are no intervening unit operations between the first and second TFF unit operations);
and wherein, following operation of the first and second TFF unit operations,
(a) the concentration of HCP in the solution containing the immunoglobulin is reduced by at least 10% relative to the HCP concentration in the cell culture supernatant; and/or
(b) the concentration of HCP in the solution containing the immunoglobulin is 400,000 ng per mg of the immunoglobulin or less; and/or
(c) the concentration of HCDNA in the solution containing the immunoglobulin is reduced by at least 30% relative to the HCDNA concentration in the cell culture supernatant; and/or
(d) the concentration of HCDNA in the solution containing the immunoglobulin is 1,500,000 pg per mg of immunoglobulin or less.

In other aspects, the invention encompasses methods of separating an immunoglobulin (*e.g.*, antibody) of interest from a cell culture supernatant comprising the immunoglobulin using dual stage TFF, wherein the dual stage TFF comprises a first TFF unit operation and a second TFF unit operation,
wherein
(i) said first TFF unit operation filters a first product stream containing the immunoglobulin of interest using a first ultrafiltration membrane having a cut-off value of 50 kD or less, preferably 50 kD; and
(ii) said second TFF unit operation filters a second product stream containing the immunoglobulin of interest using a second ultrafiltration membrane having a cut-off value of between 300 kD and 1000 kD, preferably 300 kD;
wherein
the first product steam is a cell culture supernatant that has been sterilized by filtration through a filter having a pore size of between 0.1 µm and 0.45 µm (preferably 0.2 µm or 0.22 µm);
wherein, following operation of the first and second TFF unit operations,
(a) the concentration of HCP in the solution containing the immunoglobulin is reduced by at least 10% relative to the HCP concentration in the cell culture supernatant; and/or
(b) the concentration of HCP in the solution containing the immunoglobulin is 400,000 ng per mg of the immunoglobulin or less; and/or
(c) the concentration of HCDNA in the solution containing the immunoglobulin is reduced by at least 30% relative to the HCDNA concentration in the cell culture supernatant; and/or
(d) the concentration of HCDNA in the solution containing the immunoglobulin is 1,500,000 pg per mg of immunoglobulin or less;
and wherein
said methods further comprise a chromatography process (*e.g.,* an ion exchange chromatography process and/or an affinity chromatography process) downstream of said first and said second TFF unit operations.

In other aspects, the invention encompasses methods of separating an immunoglobulin (*e.g*., antibody) of interest from a cell culture supernatant comprising the immunoglobulin using dual stage TFF, wherein the dual stage TFF comprises a first TFF unit operation and a second TFF unit operation,
wherein
(i) said first TFF unit operation filters a first product stream containing the immunoglobulin of interest using a first ultrafiltration membrane having a cut-off value of 50 kD or less, preferably 50 kD; and
(ii) said second TFF unit operation filters a second product stream containing the immunoglobulin of interest using a second ultrafiltration membrane having a cut-off value of between 300 kD and 1000 kD, preferably 300 kD;
wherein
the second TFF unit operation is operated directly downstream of the first TFF unit operation (*i.e.,* in this aspect, there are no intervening unit operations between the first and second TFF unit operations);
wherein, following operation of the first and second TFF unit operations,
(a) the concentration of HCP in the solution containing the immunoglobulin is reduced by at least 10% relative to the HCP concentration in the cell culture supernatant; and/or
(b) the concentration of HCP in the solution containing the immunoglobulin is 400,000 ng per mg of the immunoglobulin or less; and/or
(c) the concentration of HCDNA in the solution containing the immunoglobulin is reduced by at least 30% relative to the HCDNA concentration in the cell culture supernatant; and/or
(d) the concentration of HCDNA in the solution containing the immunoglobulin is 1,500,000 pg per mg of immunoglobulin or less;
and wherein
said methods further comprise a chromatography process (*e.g.*, an ion exchange chromatography process and/or an affinity chromatography process) downstream of said first and said second TFF unit operations.

In other aspects, the invention encompasses methods of separating an immunoglobulin (*e.g.*, antibody) of interest from a cell culture supernatant comprising the immunoglobulin using dual stage TFF, wherein the dual stage TFF comprises a first TFF unit operation and a second TFF unit operation,
wherein
(i) said first TFF unit operation filters a first product stream containing the immunoglobulin of interest using a first ultrafiltration membrane having a cut-off value of 50 kD or less, preferably 50 kD; and
(ii) said second TFF unit operation filters a second product stream containing the immunoglobulin of interest using a second ultrafiltration membrane having a cut-off value of between 300 kD and 1000 kD, preferably 300 kD;
wherein
the first product steam is a cell culture supernatant that has been sterilized by filtration through a filter having a pore size of between 0.1 µm and 0.45 µm (preferably 0.2 µm or 0.22 µm);
wherein
the second TFF unit operation is operated directly downstream of the first TFF unit operation (*i.e.,* in this aspect, there are no intervening unit operations between the first and second TFF unit operations);
wherein, following operation of the first and second TFF unit operations,
(a) the concentration of HCP in the solution containing the immunoglobulin is reduced by at least 10% relative to the HCP concentration in the cell culture supernatant; and/or
(b) the concentration of HCP in the solution containing the immunoglobulin is 400,000 ng per mg of the immunoglobulin or less; and/or
(c) the concentration of HCDNA in the solution containing the immunoglobulin is reduced by at least 30% relative to the HCDNA concentration in the cell culture supernatant; and/or
(d) the concentration of HCDNA in the solution containing the immunoglobulin is 1,500,000 pg per mg of immunoglobulin or less;
and wherein
said methods further comprise a chromatography process (*e.g*., an ion exchange chromatography process and/or an affinity chromatography process) downstream of said first and said second TFF unit operations.

In certain aspects, the invention encompasses methods of separating an immunoglobulin (*e.g.*, antibody) of interest from a cell culture supernatant comprising the immunoglobulin using dual stage TFF, wherein the dual stage TFF comprises a first TFF unit operation, a second TFF unit operation and a third TFF unit operation, and wherein
(i) said first TFF unit operation filters a first product stream containing the immunoglobulin of interest using a first ultrafiltration membrane having a cut-off value of 50 kD or less, preferably 50 kD;
(ii) said second TFF unit operation filters a second product stream containing the immunoglobulin of interest using a second ultrafiltration membrane having a cut-off value of between 300 kD and 1000 kD, preferably 300 kD; and
(iii) said third TFF unit operation filters a third product stream containing the immunoglobulin of interest using a third ultrafiltration membrane having a cut-off value of 50 kD or less, preferably 50 kD;
and wherein, following operation of the first, second and third TFF unit operations,
(a) the concentration of HCP in the solution containing the immunoglobulin is reduced by at least 10% relative to the HCP concentration in the cell culture supernatant; and/or
(b) the concentration of HCP in the solution containing the immunoglobulin is 400,000 ng per mg of the immunoglobulin or less; and/or
(c) the concentration of HCDNA in the solution containing the immunoglobulin is reduced by at least 30% relative to the HCDNA concentration in the cell culture supernatant; and/or
(d) the concentration of HCDNA in the solution containing the immunoglobulin is 1,500,000 pg per mg of immunoglobulin or less.

In other aspects, the invention encompasses methods of separating an immunoglobulin (*e.g*., antibody) of interest from a cell culture supernatant comprising the immunoglobulin using dual stage TFF, wherein the dual stage TFF comprises a first TFF unit operation, a second TFF unit operation and a third TFF unit operation,
wherein
(i) said first TFF unit operation filters a first product stream containing the immunoglobulin of interest using a first ultrafiltration membrane having a cut-off value of 50 kD or less, preferably 50 kD;
(ii) said second TFF unit operation filters a second product stream containing the immunoglobulin of interest using a second ultrafiltration membrane having a cut-off value of of between 300 kD and 1000 kD, preferably 300 kD; and
(iii) said third TFF unit operation filters a third product stream containing the immunoglobulin of interest using a third ultrafiltration membrane having a cut-off value of 50 kD or less, preferably 50 kD;
wherein
the second TFF unit operation is operated directly downstream of the first TFF unit operation *(i.e.,* in this aspect, there are no intervening unit operations between the first and second TFF unit operations);
wherein
the third TFF unit operation is operated directly downstream or in parallel with the second TFF unit operation (*i.e.,* in this aspect, there are no intervening unit operations between the second and third TFF unit operations);
and wherein, following operation of the first, second and third TFF unit operations,
(a) the concentration of HCP in the solution containing the immunoglobulin is reduced by at least 10% relative to the HCP concentration in the cell culture supernatant; and/or
(b) the concentration of HCP in the solution containing the immunoglobulin is 400,000 ng per mg of the immunoglobulin or less; and/or
(c) the concentration of HCDNA in the solution containing the immunoglobulin is reduced by at least 30% relative to the HCDNA concentration in the cell culture supernatant; and/or
(d) the concentration of HCDNA in the solution containing the immunoglobulin is 1,500,000 pg per mg of immunoglobulin or less.

In other aspects, the invention encompasses methods of separating an immunoglobulin (*e.g.*, antibody) of interest from a cell culture supernatant comprising the immunoglobulin using dual stage TFF, wherein the dual stage TFF comprises a first TFF unit operation, a second TFF unit operation and a third TFF unit operation, wherein
(i) said first TFF unit operation filters a first product stream containing the immunoglobulin of interest using a first ultrafiltration membrane having a cut-off value of 50 kD or less, preferably 50 kD;
(ii) said second TFF unit operation filters a second product stream containing the immunoglobulin of interest using a second ultrafiltration membrane having a cut-off value of of between 300 kD and 1000 kD, preferably 300 kD; and
(iii) said third TFF unit operation filters a third product stream containing the immunoglobulin of interest using a third ultrafiltration membrane having a cut-off value of 50 kD or less, preferably 50 kD;
wherein
the first product steam is a cell culture supernatant that has been sterilized by filtration through a filter having a pore size of between 0.1 µm and 0.45 µm (preferably 0.2 µm or 0.22 µm);
and wherein, following operation of the first, second and third TFF unit operations,
(a) the concentration of HCP in the solution containing the immunoglobulin is reduced by at least 10% relative to the HCP concentration in the cell culture supernatant; and/or
(b) the concentration of HCP in the solution containing the immunoglobulin is 400,000 ng per mg of the immunoglobulin or less; and/or
(c) the concentration of HCDNA in the solution containing the immunoglobulin is reduced by at least 30% relative to the HCDNA concentration in the cell culture supernatant; and/or
(d) the concentration of HCDNA in the solution containing the immunoglobulin is 1,500,000 pg per mg of immunoglobulin or less.

In other aspects, the invention encompasses methods of separating an immunoglobulin (*e.g*., antibody) of interest from a cell culture supernatant comprising the immunoglobulin using dual stage TFF, wherein the dual stage TFF comprises a first TFF unit operation, a second TFF unit operation and a third TFF unit operation,
wherein
(i) said first TFF unit operation filters a first product stream containing the immunoglobulin of interest using a first ultrafiltration membrane having a cut-off value of 50 kD or less, preferably 50 kD;
(ii) said second TFF unit operation filters a second product stream containing the immunoglobulin of interest using a second ultrafiltration membrane having a cut-off value of of between 300 kD and 1000 kD, preferably 300 kD; and
(iii) said third TFF unit operation filters a third product stream containing the immunoglobulin of interest using a third ultrafiltration membrane having a cut-off value of 50 kD or less, preferably 50 kD;
wherein, following operation of the first, second and third TFF unit operations,
(a) the concentration of HCP in the solution containing the immunoglobulin is reduced by at least 10% relative to the HCP concentration in the cell culture supernatant; and/or
(b) the concentration of HCP in the solution containing the immunoglobulin is 400,000 ng per mg of the immunoglobulin or less; and/or
(c) the concentration of HCDNA in the solution containing the immunoglobulin is reduced by at least 30% relative to the HCDNA concentration in the cell culture supernatant; and/or
(d) the concentration of HCDNA in the solution containing the immunoglobulin is 1,500,000 pg per mg of immunoglobulin or less;
and wherein
said methods further comprise a chromatography process (*e.g.*, an ion exchange chromatography process and/or an affinity chromatography process) downstream of said first, said second and said third TFF unit operations.

In other aspects, the invention encompasses methods of separating an immunoglobulin (*e.g.*, antibody) of interest from a cell culture supernatant comprising the immunoglobulin using dual stage TFF, wherein the dual stage TFF comprises a first TFF unit operation, a second TFF unit operation and a third TFF unit operation,
wherein
(i) said first TFF unit operation filters a first product stream containing the immunoglobulin of interest using a first ultrafiltration membrane having a cut-off value of 50 kD or less, preferably 50 kD;
(ii) said second TFF unit operation filters a second product stream containing the immunoglobulin of interest using a second ultrafiltration membrane having a cut-off value of of between 300 kD and 1000 kD, preferably 300 kD; and
(iii) said third TFF unit operation filters a third product stream containing the immunoglobulin of interest using a third ultrafiltration membrane having a cut-off value of 50 kD or less, preferably 50 kD;
wherein
the second TFF unit operation is operated directly downstream of the first TFF unit operation (*i.e.,* in this aspect, there are no intervening unit operations between the first and second TFF unit operations);
wherein
the third TFF unit operation is operated directly downstream or in parallel with the second TFF unit operation (*i.e*., in this aspect, there are no intervening unit operations between the second and third TFF unit operations);
wherein
the first product steam is a cell culture supernatant that has been sterilized by filtration through a filter having a pore size of between 0.1 µm and 0.45 µm (preferably 0.2 µm or 0.22 µm);
and wherein, following operation of the first, second and third TFF unit operations,
(a) the concentration of HCP in the solution containing the immunoglobulin is reduced by at least 10% relative to the HCP concentration in the cell culture supernatant; and/or
(b) the concentration of HCP in the solution containing the immunoglobulin is 400,000 ng per mg of the immunoglobulin or less; and/or
(c) the concentration of HCDNA in the solution containing the immunoglobulin is reduced by at least 30% relative to the HCDNA concentration in the cell culture supernatant; and/or
(d) the concentration of HCDNA in the solution containing the immunoglobulin is 1,500,000 pg per mg of immunoglobulin or less.

In other aspects, the invention encompasses methods of separating an immunoglobulin (*e.g*., antibody) of interest from a cell culture supernatant comprising the immunoglobulin using dual stage TFF, wherein the dual stage TFF comprises a first TFF unit operation, a second TFF unit operation and a third TFF unit operation,
wherein
(i) said first TFF unit operation filters a first product stream containing the immunoglobulin of interest using a first ultrafiltration membrane having a cut-off value of 50 kD or less, preferably 50 kD;
(ii) said second TFF unit operation filters a second product stream containing the immunoglobulin of interest using a second ultrafiltration membrane having a cut-off value of of between 300 kD and 1000 kD, preferably 300 kD; and
(iii) said third TFF unit operation filters a third product stream containing the immunoglobulin of interest using a third ultrafiltration membrane having a cut-off value of 50 kD or less, preferably 50 kD;
wherein
the second TFF unit operation is operated directly downstream of the first TFF unit operation (*i.e.,* in this aspect, there are no intervening unit operations between the first and second TFF unit operations);
wherein
the third TFF unit operation is operated directly downstream or in parallel with the second TFF unit operation (*i.e.,* in this aspect, there are no intervening unit operations between the second and third TFF unit operations);
wherein, following operation of the first, second and third TFF unit operations,
(a) the concentration of HCP in the solution containing the immunoglobulin is reduced by at least 10% relative to the HCP concentration in the cell culture supernatant; and/or
(b) the concentration of HCP in the solution containing the immunoglobulin is 400,000 ng per mg of the immunoglobulin or less; and/or
(c) the concentration of HCDNA in the solution containing the immunoglobulin is reduced by at least 30% relative to the HCDNA concentration in the cell culture supernatant; and/or
(d) the concentration of HCDNA in the solution containing the immunoglobulin is 1,500,000 pg per mg of immunoglobulin or less;
and wherein
said methods further comprise a chromatography process (*e.g*., an ion exchange chromatography process and/or an affinity chromatography process) downstream of said first, said second and said third TFF unit operations.

In other aspects, the invention encompasses methods of separating an immunoglobulin (*e.g.*, antibody) of interest from a cell culture supernatant comprising the immunoglobulin using dual stage TFF, wherein the dual stage TFF comprises a first TFF unit operation, a second TFF unit operation and a third TFF unit operation,
wherein
(i) said first TFF unit operation filters a first product stream containing the immunoglobulin of interest using a first ultrafiltration membrane having a cut-off value of 50 kD or less, preferably 50 kD;
(ii) said second TFF unit operation filters a second product stream containing the immunoglobulin of interest using a second ultrafiltration membrane having a cut-off value of of between 300 kD and 1000 kD, preferably 300 kD; and
(iii) said third TFF unit operation filters a third product stream containing the immunoglobulin of interest using a third ultrafiltration membrane having a cut-off value of 50 kD or less, preferably 50 kD;
wherein
the first product steam is a cell culture supernatant that has been sterilized by filtration through a filter having a pore size of between 0.1 µm and 0.45 µm (preferably 0.2 µm or 0.22 µm);
wherein, following operation of the first, second and third TFF unit operations,
(a) the concentration of HCP in the solution containing the immunoglobulin is reduced by at least 10% relative to the HCP concentration in the cell culture supernatant; and/or
(b) the concentration of HCP in the solution containing the immunoglobulin is 400,000 ng per mg of the immunoglobulin or less; and/or
(c) the concentration of HCDNA in the solution containing the immunoglobulin is reduced by at least 30% relative to the HCDNA concentration in the cell culture supernatant; and/or
(d) the concentration of HCDNA in the solution containing the immunoglobulin is 1,500,000 pg per mg of immunoglobulin or less.
and wherein
said methods further comprise a chromatography process (*e.g*., an ion exchange chromatography process and/or an affinity chromatography process) downstream of said first, said second and said third TFF unit operations.

In other aspects, the invention encompasses methods of separating an immunoglobulin (*e.g.*, antibody) of interest from a cell culture supernatant comprising the immunoglobulin using dual stage TFF, wherein the dual stage TFF comprises a first TFF unit operation, a second TFF unit operation and a third TFF unit operation,
wherein
(i) said first TFF unit operation filters a first product stream containing the immunoglobulin of interest using a first ultrafiltration membrane having a cut-off value of 50 kD or less, preferably 50 kD;
(ii) said second TFF unit operation filters a second product stream containing the immunoglobulin of interest using a second ultrafiltration membrane having a cut-off value of of between 300 kD and 1000 kD, preferably 300 kD; and
(iii) said third TFF unit operation filters a third product stream containing the immunoglobulin of interest using a third ultrafiltration membrane having a cut-off value of 50 kD or less, preferably 50 kD;
wherein
the second TFF unit operation is operated directly downstream of the first TFF unit operation (*i.e.,* in this aspect, there are no intervening unit operations between the first and second TFF unit operations);
wherein
the third TFF unit operation is operated directly downstream or in parallel with the second TFF unit operation (*i.e.,* in this aspect, there are no intervening unit operations between the second and third TFF unit operations);
wherein
the first product steam is a cell culture supernatant that has been sterilized by filtration through a filter having a pore size of between 0.1 µm and 0.45 µm (preferably 0.2 µm or 0.22 µm);
wherein, following operation of the first, second and third TFF unit operations,
(a) the concentration of HCP in the solution containing the immunoglobulin is reduced by at least 10% relative to the HCP concentration in the cell culture supernatant; and/or
(b) the concentration of HCP in the solution containing the immunoglobulin is 400,000 ng per mg of the immunoglobulin or less; and/or
(c) the concentration of HCDNA in the solution containing the immunoglobulin is reduced by at least 30% relative to the HCDNA concentration in the cell culture supernatant; and/or
(d) the concentration of HCDNA in the solution containing the immunoglobulin is 1,500,000 pg per mg of immunoglobulin or less;
and wherein
said methods further comprise a chromatography process (*e.g.,* an ion exchange chromatography process and/or an affinity chromatography process) downstream of said first, said second and said third TFF unit operations.
Any of the dual stage TFF methods disclosed herein, whether described as an aspect and/or embodiment of the invention, and whether described as preferred or not, can be combined with downstream standard purification processes known in the art such as affinity purification or, in particular, with downstream purification processes that are not based on affinity isolation, e.g., ion exchange chromatography processes. In a specific example, any of the dual stage TFF methods disclosed herein can be upstream of an affinity purification process comprising, e.g., the use of Protein A (*e.g.,* Protein A chromatography). In other examples, any of the dual stage TFF methods disclosed herein can be upstream of non-affinity purification processes comprising, *e.g*., chromatography processes/unit operations such as CEX and/or AEX. In particular embodiments, the dual stage TFF methods can be upstream of non-affinity purification processes comprising both CEX and AEX, wherein the AEX is optionally upstream of the CEX.
In certain embodiments, the dual stage TFF methods are upstream of a CEX process/unit operation wherein the pH of the solution containing the molecule of interest is adjusted to 4.0, 4.5, 5.0, 5.5, 6.0, 6.5 or 7.0 prior to the CEX process/unit operation. In a particular aspect of this embodiment, adjustment of the pH prior to CEX according to the methods of the invention is not associated with the precipitation of HCP and/or HCDNA within the solution. In certain aspects according to this embodiment, the dual stage TFF methods are upstream of a CEX process/unit operation, wherein a precipitation or precipitate filtration process is not implemented prior to the CEX process/unit operation.
Other objects and advantages of this invention will become apparent from the following description wherein are set forth, by way of illustration and example, certain embodiments of this invention.

### DEFINITIONS

In general, the following words or phrases have the indicated definition when used in the summary, description, examples, and claims.
As used herein, the term "about" in connection with a number indicates ± 5% of the number. When used in connection with a measurement performed by a device (*e.g.*, pH as determined by a pH meter) or performed according to a standard method known in the art (*e.g*., protein concentration of a solution determined by HPLC, UV adsorption, ELISA, standard kits (*e.g.,* colorometric assay)), it indicates a number within the standard error known for such a device or within one standard deviation of the determined value for such a method.

The term "antibody" is used in the broadest sense and specifically covers, for example, single monoclonal antibodies (including agonist, antagonist, and neutralizing antibodies; as well as de-immunized, murine, chimeric, humanized and human antibodies), antibody compositions with poly-epitopic specificity, single-chain antibodies, diabodies, triabodies, immuno-conjugates, synthetic antibodies, camelized antibodies, single-chain Fvs (scFv), single chain antibodies, Fab fragments, F(ab') fragments, F(ab')₂ fragments, disulfide-linked Fvs (sdFv), intrabodies, and epitope-binding fragments of any of the above. In particular, antibodies include immunoglobulin molecules and immunologically active fragments of immunoglobulin molecules, *i.e.,* molecules that contain an antigen binding site. The term "antibody" also encompasses immunoglobulin molecules of any type (*e.g.,* IgG, IgE, IgM, IgD, IgA and IgY), class (*e.g.,* IgG₁, IgG₂, IgG₃, IgG₄, IgA₁ and IgA₂) or subclass.

As used herein, the term "between" in connection with the definition of a range expressly includes the endpoints of that range. Thus if the parameter is defined herein as being "between X and Y", it is expressly intended that the parameter may have a value equal to X or greater than X, so long as the value is at most Y, *i.e.,* is not more than Y. In other words, the values defined by the phrase "between X and Y" and analogous constructions expressly includes the values X and Y. For example, as used throughout this disclosure, the phrase, "wherein the membrane has a cut-off value of between 300 kD and 100 kD" is expressly intended to encompass embodiments wherein the membrane has a cut-off value of 300 kD as well as embodiments wherein the membrane has a cut-off value or 1000 kD.

As used herein, the expressions "cell", "cell line", and "cell culture" are used interchangeably, and all such designations include progeny. In particular, the invention encompasses the separation and/or purification of molecules of interest, *e.g*., proteins, from the products of cells, cell lines and cell cultures. Such products typically include conditioned cell media and/or lysed and homogenized cells and cell cultures (*e.g*., homogenized cells and cell components within conditioned cell media). The methods of the invention are particularly suited to the processing of products from transgenic cells, cell lines and cell cultures, wherein the transgenic cells, cell lines and cell cultures express the molecule of interest. In preferred embodiments, the invention encompasses the use of clarified, conditioned cell culture media.

It is understood that the methods of the invention relate to the separation, purification and/or processing of a molecule of interest, *e.g.,* a protein, from a solution containing the molecule. As such, where the solution containing the molecule of interest is cell culture media or a fractionated or clarified part of cell culture media, it is understood that such media is necessarily conditioned cell culture media (so as to comprise the molecule of interest). Therefore, as used herein, the term "cell culture solution" and analogous terms refer to any solution of a biological process or system expected to contain the molecule of interest, including but not limited to, *e.g.,* conditioned cell culture supernatant; clarified conditioned cell culture supernatant; clarified, homogenized/lysed cell cultures, etc. In preferred embodiments of the invention, the feed stream of the dual stage TFF is a cell culture supernatant as defined herein.

The dual stage TFF methods disclosed herein also encompass the optional processing of a cell culture media for clarification and/or sterilization upstream of the first TFF unit operation, *i.e.,* prior to operation of the dual stage TFF unit operations described in this disclosure. As used herein, the term "clarified" and "clarification" refer to the removal of particulate matter from a solution, including filtration sterilization. The term "sterilized" as used herein in understood to be used in connection with a solution containing proteins; accordingly, "sterilization" of such solutions is understood to be effected preferably by filtration and not by heat to avoid protein denaturation and/or protein aggregation. A "clarified"/"sterilized" solution with reference to any cell culture media encompassed by the methods of the invention is a solution that has been filtered through a membrane of between 0.1 µm and 0.45 µm, preferably a filter that has a pore size of 0.2 µm or 0.22 µm.

As used herein, the term "dual stage tangential flow ultrafiltration", "dual stage TFF" and analogous terms refers to the use of at least two TFF and/or diafiltration/TFF unit operations in combination. In certain embodiments, the term "dual stage TFF" refers to the use of three or more TFF and/or diafiltration/TFF unit operations in combination. The use of the term "in combination" does not restrict the order in which the solution containing the molecule of interest (*e.g.*, protein) proceeds through the two or more TFF and/or diafiltration/TFF unit operations. The term "in combination" also does not restrict the process method to the use of the at least two TFF and/or diafiltration/TFF unit operations in immediate sequence (*i.e.,* one unit operation immediately following the other); therefore, dual stage tangential flow filtration encompasses processes comprising one or more unit operations that are not TFF and/or diafiltration/TFF interspersed between the at least two TFF and/or diafiltration/TFF unit operations explicitly defined by the methods described herein. The use of the term "in combination" also does not restrict the timing of the individual two or more TFF and/or diafiltration/TFF unit operations described herein, and the two or more individual unit operations may be separated by 1 minute, 5 minutes, 15 minutes, 30 minutes, 45 minutes, 1 hour, 2 hours, 4 hours, 6 hours, 12 hours, 24 hours, 48 hours or 72 hours. Further, the two or more individual unit operations may proceed concomitantly with one another, e.g., be run in parallel as is known in the art. The term "dual-stage TFF" may also refer to the use of at least two TFF and/or diafiltration/TFF unit operations in combination with standard purification protocols or processes as known in the art, *e.g.,* CEX and AEX.

As used in the context of the methods disclosed herein, the expression "crude feed stream" typically refers to the raw material or raw solution derived from a production scheme that is delivered to the initial unit operation, which raw material contains the molecule of interest (*e.g*., biomolecule, protein, polypeptide, antibody, etc.) and may further contain various contaminants (*e.g*., non-desired proteins, cell fragments, viruses, DNA, etc). In contrast, "product stream" is typically used in a more general context to refer to a solution containing the molecule of interest that may or may not have already been subject to some processing (*e.g*., one or more unit operations including clarification, filtration, etc.). However for purposes of the methods of the invention, any distinction between "feed stream" and "product stream" is irrelevant, as both terms relate simply to solutions containing the product of interest (*e.g.*, biomolecule, protein, polypeptide, antibody, etc.), which solutions may generally be processed according to the methods of the invention regardless of whether prior upstream processing has occurred. If any distinction is necessary between "feed stream" and "product stream" is necessary for the purposes of this disclosure, it will be readily apparent to one of skill in the art from context.

As used herein, the terms "microfiltration" and "ultrafiltration" reference filtration parameters as commonly understood in the art. In particular, the term microfiltration commonly refers to the use of a filtration membrane with a pore size between 0.1 and 10 µm, and the term ultrafiltration refers to the use of a filtration membrane with a pore size of between 0.001 and 0.1 µm. Microfiltration is typically used for clarification, sterilization, removal of microparticulates, and for cell harvests; ultrafiltration is typically used for separating and concentrating dissolved molecules (*e.g.*, protein, peptides, nucleic acids, carbohydrates, and other biomolecules), for exchange buffers, and for gross fractionation. The methods of the present invention are directed to ultrafiltration using TFF and diafiltration/TFF unit operations. In certain embodiments, the dual stage TFF methods disclosed herein may also comprise the use of ultrafiltration membranes or membranes with even larger pore sizes in, *e.g.,* one of more diafiltration units, for unit operations such as sterilization prior to, *i.e*., upstream, of the initial/first TFF unit operation described herein. Sterilization of load fluids, *e.g.,* cell culture supernatants containing proteins of interest, via filtration methods are well known in the art and typically comprise filtration of the supernatant through filter membranes having pore sizes ranging from about 0.1 µm to about 0.45 µm. Accordingly, the present invention also encompasses dual stage TFF methods comprising the sterilization of a cell culture supernatant prior to operation of the initial/first TFF unit operation, wherein the sterilization is effected by filtration through a suitable filter membrane having a pore size of between 0.1 µm and 0.45 µm, preferably a filter having a pore size of 0.2 or 0.22 µm.
As used herein, the expression "molecule of interest" and analogous expressions refers to a molecule that is to be separated from a solution or suspension. The molecules of interest are separated from other particles or molecules in the solution or suspension based on the size of the molecule of interest. In some embodiments of the methods disclosed herein, the molecules of interest are also separated from the original fluid component of the solution or suspension by a process of buffer exchange via, *e.g.,* a diafiltration unit operation. Separation of the molecules of interest from contaminants and/or other undesired components of the solution or suspension may also be referenced as "purification". In preferred embodiments, the molecules of interest are biomolecules. That is, the molecules of interest are of biological or biochemical origin and/or are produced by mammalian or microorganism (*e.g*., bacteria, fungi yeast) cell cultures, which cell cultures may or may not be transgenic. Therefore, the molecule of interest, *e.g.*, protein, may be a molecule natively produced by a cell culture or may be a recombinant product. The molecules of interest may also be produced *in vivo* using animal models, *e.g.,* transgenic animals, or may be produced by *in vitro* processes. The *in vitro* processes may be based on the biochemical pathways found in mammalian and/or microorganism cells. Examples of molecules of interest in the context of the present methods include proteins, peptides, polypeptides, antibodies, enzymes and fragments thereof.

### BRIEF DESCRIPTION OF THE FIGURES

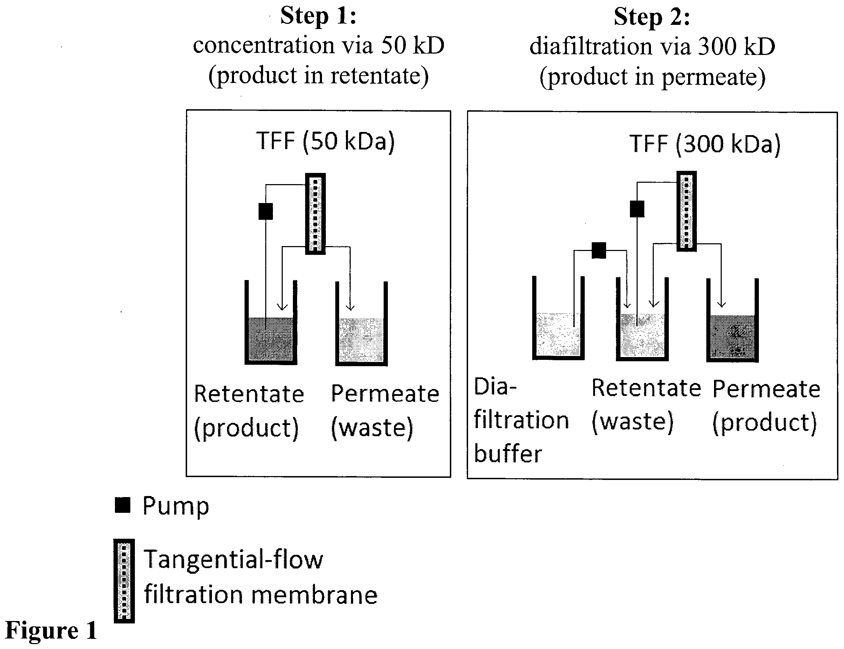

- **Figure 1**: is a schematic of a dual stage TFF process for the separation of a protein having a molecular weight of 150 ± 30 kD (*e.g.*, an antibody) having a TFF/concentration unit operation comprising an ultrafiltration membrane having a 50 kD cut-off value, followed by a TFF/diafiltration unit operation having comprising an ultrafiltration membrane having a 300 kD cut-off.

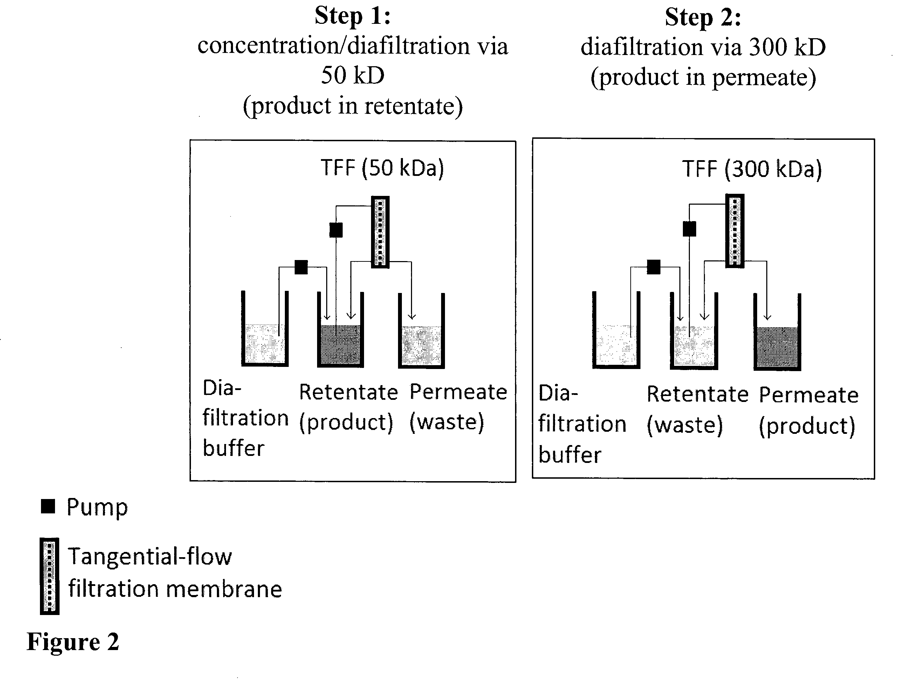

- **Figure** 2: is a schematic of a dual stage TFF process for the separation of a protein having a molecular weight of 150 ± 30 kD (*e.g.,* an antibody) having a TFF/diafiltration unit operation comprising an ultrafiltration membrane having a 50 kD cut-off value, followed by a TFF/diafiltration unit operation having comprising an ultrafiltration membrane having a 300 kD cut-off.

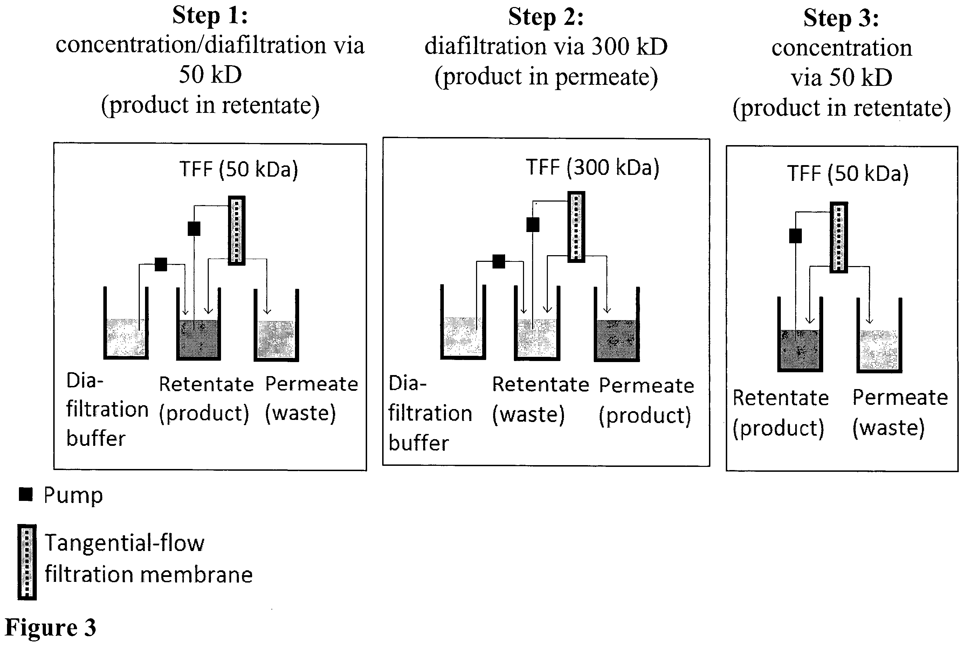

- **Figure** 3: is a schematic of a dual stage TFF process for the separation of a protein having a molecular weight of 150 ± 30 kD (*e.g.,* an antibody) having a TFF/concentration/diafiltration unit operation comprising an ultrafiltration membrane having a 50 kD cut-off value, followed by a TFF/diafiltration unit operation comprising an ultrafiltration membrane having a 300 kD cut-off, which is followed a third TFF/conccntration unit operation having an ultrafiltration membrane having a 50 kD cut-off value.

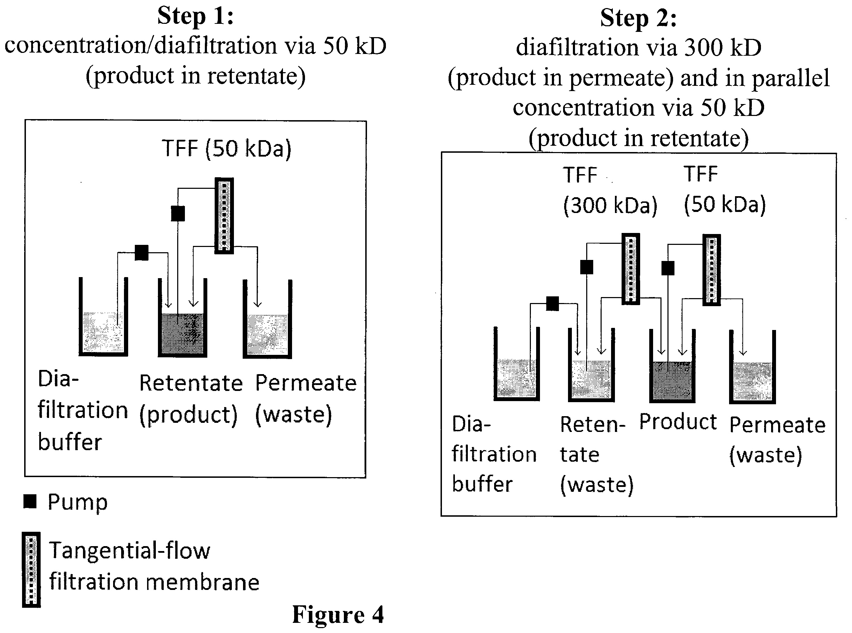

- **Figure** 4: is a schematic of a dual stage TFF process for the separation of a protein having a molecular weight of 150 ± 30 kD (*e.g.,* an antibody) having a TFF/concentration/diafiltration unit operation comprising an ultrafiltration membrane having a 50 kD cut-off value, followed by a TFF/diafiltration unit operation comprising an ultrafiltration membrane having a 300 kD cut-off that is connected in parallel to a third TFF/concentration unit operation having an ultrafiltration membrane having a 50 kD cut-off value.

### DETAILED DESCRIPTION

It has surprisingly been discovered that the use of dual stage tangential flow ultrafiltration ("dual stage TFF") to separate a molecule of interest from a solution containing the molecule dramatically reduces the level of contaminants in the solution relative to standard purification processes. As such the disclosed dual stage TFF methods disclosed herein may supplement existing purification schemes and/or replace one or more unit operations within an existing purification schemes. In particular, the dual stage TFF methods disclosed herein reduce contaminant levels to such an extent that downstream processing and/or purification of the molecule can proceed without the need for additional impurity precipitation and clarification/precipitate filtration processes. The methods disclosed herein are of particular use in separation and/or purification processes for biomolecules. The dual-stage TFF methods disclosed herein may be used in any process for the separation/purification/isolation of a molecule of interest from a solution. The substantial reduction of contaminant/impurity levels afforded by the dual-stage TFF methods disclosed herein are also of relevance to any process wherein contaminant/impurity levels negatively affect process performance and/or unit operation function. For example, the dual-stage TFF method may be used upstream of an affinity chromatography, *e.g.,* a protein A column, or ion exchange chromatography unit operations in order to protect (via a reduced impurity burden), for example, the affinity molecule, *e.g.,* protein A, or the chromatography resin in order to prolong its effective working life. In certain embodiments, the dual-stage TFF methods disclosed herein may replace one or more unit operations in existing purification schemes, *e.g.,* affinity chromatography, and, thus, may be used in the non-affinity purification/isolation/separation of a biomolecule, *e.g.,* an antibody, from a solution containing the biomolecule. In other embodiments, the dual-stage TFF methods disclosed herein may be used in processes also comprising affinity chromatography, *e.g.,* protein A chromatography.

The dual-stage TFF methods disclosed herein reduce contaminant/impurity levels such that downstream purification processes can proceed without the use of an intermediate precipitation and clarification process, *e.g.,* precipitation and clarification process, *e.g.,* prior to cation exchange chromatography (CEX). In certain embodiments, the dual-stage TFF methods may also replace one or more unit operations of traditional purification processes, *e.g.,* affinity chromatography. Accordingly, methods of the invention may result in substantial savings of both the direct costs and indirect costs associated with any production, processing and/or purification stream. With respect to the direct costs, the dual stage TFF methods of the invention may, in certain embodiments, replace affinity based chromatography methods. Because filtration membranes cost substantially less that affinity-based chromatography materials (*e.g.,* to supply a typical industrial column, cost at least ¼ less) and exhibit longer operational life times, the use of the membrane based dual stage TFF methods may directly save material costs for this initial purification step. Moreover, the use of affinity chromatography is also associated with a host of indirect costs, including careful preparation of load material to ensure compatibility with the affinity material; monitoring of affinity material leeching; and process shut down times to regenerate affinity material. Further, because the parameters of the binding and/or elution buffers for the affinity chromatography may be incompatible with downstream processes, the product stream may require further unit operations such as buffer exchange prior to common additional downstream purification/polishing steps such as ion exchange chromatography. Each of these additional costs would be indirectly saved by use of the dual stage TFF methods of the invention. The dual stage TFF methods of the present invention are based on size, which membrane performance is not as sensitive to feed stream parameters and do not require monitoring because leeching cannot occur. Further, it is possible to process/purify the molecule of interest in the feed stream while concurrently processing the stream solution parameters such that any output stream is fully compatible with downstream processes, *e.g.,* CEX, with minimal additional processing.

Methods for the separation of a molecule of interest from a solution containing the molecule based on its size, *e.g.,* molecular weight, are known. The most widely practiced of such methods are membrane filtration processes. Currently, there are two main membrane filtration methods: Single Pass or Direct Flow Filtration (DFF) and Crossflow or Tangential Flow Filtration (TFF). The dual stage TFF methods disclosed herein relate specifically to TFF. TFF is an ultrafiltration system that is distinguished from DFF in that the flow of the solution to be filtered is not forced perpendicular to the membrane as in DFF, but rather proceeds parallel to the filter membrane. TFF has been designed to control the fluid flow pattern of a feed stream so as to enhance transport of the retained solute(s) away from the membrane surface and back into the bulk of the feed.

In TFF, the stream containing the molecule of interest is passed over the membrane at high velocities at a vector tangential to the plane of the membrane. During TFF a pressure differential is applied along the length of the membrane to cause the fluid and filterable solutes to flow through the filter. This is done to increase the mass-transfer co-efficient to allow for back diffusion. The fluid flowing in a direction parallel to the filter membrane also acts to clean the filter surface continuously and thereby prevents clogging. The retained solution (retentate) may be re-circulated and passed repeatedly over the membrane while that fluid which passes through the filter (permeate) is continually drawn off into a separate unit. According to the methods of the invention as described herein, depending on the cut-off value, pore size or other permeability parameter of the filter, the molecule of interest may be found in the permeate or the retentate of the individual TFF unit operation and, thus, either the permeate or retentate of any TFF unit operation may be collected for downstream processing according to the dual stage TFF methods described herein or downstream processing subsequent to the dual stage TFF methods. As understood by one of skill in the art, the operation of TFF is rarely completely efficient. That is, a small percentage of the molecules that are expected by design to be retained in the retentate of the TFF unit operation may nevertheless pass through the filter membrane to be found in the permeate; similarly a small amount of the molecules expected to pass through the membrane in the permeate of the TFF unit operation may nevertheless be retained by the membrane an remain in the retentate. Accordingly, in connection with the operation of a TFF unit operation the use of the terms "does not pass through the membrane" and "is found in the retentate" are not to be interpreted as absolute, but indicate that at least 90% of the molecule of interest applied to the TFF unit operation is recovered in the retentate. Similarly, the terms "does pass through the membrane" and "is found in the permeate" are also not to be interpreted as absolute, but indicate that at least 90% of the molecule of interest applied to the TFF unit operation is recovered in the permeate.

The use and implementation of TFF unit operations are well known in the art. TFF methods have been demonstrated to be of use in the filtration of blood components (see, *e.g.,* U.S. Pat. No. 4,888,115), in food production (*e.g.,* beer, EP-A2 0,208,450), in the purification of immunoglobulins from milk or colostrum (see, *e.g.,* U.S. Pat. No. 4,644,056) and in the separation of antiviral substances such as interferons production cell cultures (see, *e.g.,* U.S. Pat. No. 4,420,398). Accordingly, any method disclosed herein or known in the art may be used to effect the individual TFF unit operations of the methods disclosed herein. The present disclosure of the use of the dual stage TFF methods of the invention dos not encompass the processing of milk or fractionated solutions of milk, *e.g.,* casein rich or casein poor milk solutions/fractions, whey protein isolate, etc. Accordingly, the feed streams or product streams encompassed by the methods of the present invention are not milk or fractionated solutions of milk.

The dual stage TFF methods disclosed herein comprise the use of at least two TFF unit operations (*i.e.,* a first and a second TFF unit operation) that separate the molecule of interest based on size of the molecule.

The first TFF unit operation of the dual stage TFF method disclosed herein is characterized by filtering a first product stream containing the molecule of interest such that the molecule may be or is recovered in the retentate of the TFF unit operation. Retention of the molecule of interest in the retentate of the TFF unit operation is effected by selection of the ultrafiltration membrane such that it is impermeable to the molecule of interest. Although the selection may be based on actual or effective size of the molecule of interest, *e.g.,* by selecting a membrane with a cut-off value or pore size less than the actual or effective size, as is recognized in the art, membrane performance may be dependent on other factors (*e.g.,* membrane charge). Therefore, membranes having reported cut-off values or pore sizes greater than the size of the molecule of interest may nevertheless be functionally impermeable to the molecule during TFF unit operation. Accordingly, the first TFF unit operation is characterized by comprising the use of an ultrafiltration membrane having a cut-off value that is less than the molecular weight of the molecule of interest and/or is characterized in that the molecule cannot pass through the membrane during the first TFF unit operation. In specific embodiments, the ultrafiltration membrane of the first TFF unit operation has a cut-off value that is no more than one-half the molecular weight of the molecule of interest; that is, a cut-off value of 0.5 times the molecular weight of the molecule of interest or less.

The second TFF unit operation of the dual stage TFF method disclosed herein is characterized by filtering a second product stream containing the molecule of interest such that the molecule may be or is recovered in the permeate of the TFF unit operation. Recovery of the molecule of interest in the permeate of the TFF unit operation is effected by selection of the ultrafiltration membrane such that it is freely permeable to the molecule of interest. Although the selection may be based on actual or effective size of the molecule of interest, *e.g.,* by selecting a membrane with a cut-off value or pore size greater than the actual or effective size, as is recognized in the art, membrane performance may be dependent on other factors (*e.g.,* membrane charge). Therefore, the second TFF unit operation of the dual stage TFF method disclosed herein is characterized by comprising the use of an ultrafiltration membrane having a cut-off value that is greater than the molecular weight of the molecule of interest and/or that is characterized in that the molecule of interest can pass through the membrane. In certain embodiments, the ultrafiltration membrane has a cut-off value that is at least twice the molecular weight of the molecule of interest; that is, a cut-off value of 2 times the molecular weight of the molecule of interest or greater. Further, the present invention is directed to methods comprising the ultrafiltration of molecules; accordingly, the cut-off value of the second TFF unit operation is also limited by the upper size range for ultrafiltration recognized and/or accepted in the art, 1000 kD.

As detailed throughout the present disclosure, in preferred embodiments, the methods of the invention are directed to the separation of a protein, *e.g.,* immunoglobulin, from a solution containing the protein, *e.g.,* a cell culture supernatant that has optionally been clarified and/or sterilized, *e.g.,* filtered through a membrane having a pore size of between 0.1 µm and 0.45 µ, preferably filtered through a membrane having a pore size of 0.2 µm or 0.22 µm. According to the general methods of the invention, the cut-off values of the first and second TFF unit operations are selected to be less-than and greater-than the molecular weight of the molecule of interest, respectively. This is understood to, in particular, result in a first TFF unit operation wherein the molecule of interest does not pass through the filter membrane, and a second TFF unit operation wherein the molecule of interest does pass through the filter membrane. However, because filtration membrane performance can also depend on factors other than strict molecular size (*e.g.,* can depend on molecular charge) the present disclosure does not limit the criteria by which the membranes may be selected (provided that the molecule of interest does not pass through the membrane of the first TFF unit operation and does pass through the membrane of the second TFF unit operation). Thus, in certain embodiments, the cut-off values of the membranes used according to the dual stage TFF methods disclosed herein are selected based on membrane performance rather than strictly based on an asserted cut-off value (*e.g.,* according to a manufacturer's specifications). Methods to determine membrane performance and, specifically, to determine the permeability of a membrane to a molecule of interest, *e.g.,* protein, are well known and routinely implemented in the art.

It is stressed that the use of the terms "first" and "second" as identifiers of the at least two TFF unit operations of the dual stage TFF methods disclosed herein does not imply order. In other words, the presently disclosed methods do not require that the first TFF unit operation (characterized by the molecule of interest being in the retentate) be upstream of the second TFF unit operation (characterized by the molecule of interest being in the permeate). Rather the terms first and second as used herein are merely identifiers distinguishing the at least two TFF unit operations of the dual stage TFF method disclosed herein. Thus, in certain embodiments disclosed herein, the second TFF unit operation is upstream of the first unit operation. In preferred embodiments, the first TFF unit operation is upstream of the second TFF unit operation. In certain embodiments, the retentate stream of the first TFF unit operation forms the second product stream of the second TFF unit operation as disclosed herein. In other embodiments, one or more additional unit operations my be interspersed between the first and second TFF unit operations.

In certain embodiments, the dual stage TFF methods disclosed herein may also contain or comprise one or more TFF unit operations in addition to the first and second TFF unit operations described herein. The one or more additional TFF unit operations may comprise the use of an ultrafiltation membrane identical to or different from the first TFF unit operation, or may comprise the use of an ultrafiltration membrane having a different cut-off value from the first TFF unit operation, provided that the cut-off value is less than the molecular weight of the molecule of interest and/or is such that the molecule of interest cannot pass through the membrane.

In specific embodiments, the invention provides a third TFF unit operation characterized by filtering a third product stream containing the molecule of interest such that the molecule may be or is recovered in the retentate of the TFF unit operation. As such, the criteria for the operation of the third TFF unit operation according to this embodiment are the same as that for the operation of the first TFF unit operation. Therefore, the third TFF unit operation according to this embodiment is characterized by comprising the use of an ultrafiltration membrane having a cut-off value that is less than the molecular weight of the molecule of interest and/or is characterized in that the molecule cannot pass through the membrane during the third TFF unit operation. In specific embodiments, the ultrafiltration membrane of the third TFF unit operation has a cut-off value that is no more than one-half the molecular weight of the molecule of interest; that is, a cut-off value of 0.5 times the molecular weight of the molecule of interest or less.

As disclosed herein, the first and second TFF unit operation may be combined in any order. Thus, in the dual stage TFF method of the invention, the first TFF unit operation may be upstream of the second TFF unit operation, or the second TFF unit operation may be upstream of the first TFF unit operation. Further, the dual stage TFF methods described herein also encompass the use of one or more TFF unit operations in addition to the first and second TFF unit operations, *e.g.,* a third TFF unit operation, which additional TFF unit operations may be upstream, downstream or interspersed between the first and second TFF unit operations. In a preferred embodiment of the invention comprising a third TFF unit operation, the first TFF unit operation is upstream of the second TFF unit operation, which is upstream of the third TFF unit operation, wherein the third TFF unit operation is characterized by comprising the use of an ultrafiltration membrane having a cut-off value that is less than the molecular weight of the molecule of interest and/or is characterized in that the molecule cannot pass through the membrane during the third TFF unit operation. In accordance with this embodiment, the first, second and third TFF unit operations may be implemented directly following one another, or may be interspersed with one or more additional unit operations. In certain embodiments, the third TFF unit operation may be run in parallel with the second TFF unit operation or be implemented after completion of the second TFF unit operation.

The first and second unit operations and/or the second and third unit operations can be operated immediately upstream/downstream of one another. As used herein, the phrases, "operated immediately upstream", "operated directly downstream", "is immediately upstream", "is immediately downstream" and analogous phrases do not imply a chronological component; thus, *e.g.,* the operation of the first and second TFF unit operation or the second and third TFF unit operation need not occur or proceed within any specific time period. Rather the phrases indicate that the product stream is processed by no other unit operations between the first and second or between the second and third unit operations; thus, for example, the first TFF unit operation can be run in, *e.g.,* batch mode, the product stream stored, and/or transported and stored, and then at some future time, the product stream is processed by the second TFF unit operation (so long as no intervening unit operation occurred).
Although in certain embodiments the dual stage TFF methods of the invention are contemplated to replace affinity-based chromatography processes in any purification or processing scheme, the invention also contemplates purification methods comprising both the dual stage TFF process of the invention and affinity based purification. Thus, the invention contemplates the combination of dual stage TFF methods and one or more additional purification steps and/or unit operations, including affinity based chromatography, *e.g.,* protein A or protein G chromatography. Exemplary further purification steps or unit operations include methods expressly disclosed herein and/or those known in the art, such as affinity chromatography using microbial-derived proteins (*e.g.* protein A or protein G affinity chromatography as noted), ion exchange chromatography (*e.g.* cation exchange (carboxymethyl resins), anion exchange (amino ethyl resins) and mixed-mode exchange chromatography), thiophilic adsorption (*e.g.* with betamercaptoethanol and other SH ligands), hydrophobic interaction or aromatic adsorption chromatography (*e.g.* with phenyl-sepharose, aza-arenophilic resins, or m-aminophenylboronic acid), metal chelate affinity chromatography (e.g. with Ni(II)- and Cu(II)-affinity material), size exclusion chromatography, and preparative electrophoretic methods (such as gel electrophoresis, capillary electrophoresis) (Vijayalakshmi, M. A., Appl. Biochem. Biotech. 75 (1998) 93-102). Additional examples of purification steps or uit operations well known in the art include hydroxyl apatite chromatography; dialysis, affinity chromatography using an antibody to capture the protein, hydrophobic interaction chromatography (HIC), hydrophobic charge interaction chromatography (HCIC), ammonium sulphate precipitation, anion or cation exchange chromatography, ethanol precipitation; reverse phase HPLC; chromatography on silica, chromatofocusing and gel filtration.

The combination of the at least first and second TFF unit operations according to the dual stage TFF methods disclosed herein not only separates the molecule of interest from undesired components of the solution comprising the molecule of interest but also significantly reduces contaminants that may otherwise interfere with downstream processes. Thus, the dual stage TFF methods disclosed herein provide processing for a variety of input streams and result in an output product stream that may be further processed by downstream purification methods known in the art in an accelerated, efficient and economical manner. In particular, the dual stage TFF methods significantly reduce contaminant and impurities from the product stream without the use of costly unit operations such as precipitations and precipitate filtrations. Preliminarily, it is recognized in the art that precipitation processes are undesired and lead to decreased yields because it is not possible to recover the product solution from the precipitate factions. In addition to decreasing yields, precipitate and precipitate filtration unit operations result in direct cost increases due the such factors including but not limited to increased processing time (*e.g.,* the time required for precipitate formation and filtration), increased energy costs (*e.g.,* refrigeration during precipitation) and increased equipment costs (*e.g.,* large holding tanks for precipitation reactions). Accordingly, the potential elimination of downstream precipitation unit operations using the dual stage TFF unit operations of the present invention results in significant economic advantages including increasing processing yields, decreasing processing time and elimination of fixed costs (*e.g.,* associated with precipitation unit operations). Additionally, the substantial reduction in impurity/contaminant levels can offer further economic advantages to downstream processes such as chromatographic processes. For example, the contaminant reduction of the dual stage TFF methods may improve downstream column performance (*e.g.,* via improved dynamic binding, less fouling), increase processing time (*e.g.,* allow higher flow velocity) and improve column lifetime. Further, as is exemplified herein, the substantial reduction in contaminant load for downstream processes allow smaller chromatographic columns to be used without threat of overloading, resulting in greater economic advantages. Therefore, the dual stage TFF methods of the present invention offer multiple economic advantages for processes comprising their use.

In certain embodiments the molecules of interest are biomolecules produced from mammalian cells, microorganism cultures, and/or animals (all of which may or may not be transgenic), which biomolecules are separated from a culture solution or a body fluid of the animal. Alternatively, the molecules of interest may be biomolecules produced in an *in vitro* system based on biochemical reactions and pathways and are required to be separated from the components of the *in vitro* system, *e.g.,* enzymes and starting materially, used to manufacture the biomolecule. In preferred embodiments, the molecule of interest is a protein, *e.g.,* an antibody, that is to be separated from a cell culture solution (*e.g.,* a cell culture supernatant optionally filtered through a 0.22 µm membrane).
The methods disclosed herein comprise the use of TFF unit operations having ultrafiltration membranes. As defined in the art, ultrafiltration typically involves the use of membranes hiving pore sizes ranging from 0.001 to 0.1 µm. Additionally or alternatively, the ultrafiltration membranes may be rated according to cut-off value in kD, which is the maximum approximate molecular weight that can freely pass through the membrane. Ultrafiltration membranes are known to be of use in the separation of, including but not limited to, proteins, polypeptides, colloids, immunoglobulins, fusion proteins, immunoglobulin fragments, mycoplasm, endotoxins, viruses, amino acids, DNA, RNA, and carbohydrates.
The methods disclosed herein are not limited to any specific ultrafiltration membrane in that various types of membrane are known, can be commercially purchased, and/or can be produced according to methods well known in the art so that it has the desired molecular weight cut-off and does not adsorb molecule of interest (*e.g.,* protein, antibody, etc.). Examples of the ultrafiltration membranes suitable for the methods of the present invention include membranes formed from cellulose and cellulose derivatives like cellulose acetate (CA), polysulfone (PS), polyethersulfone (PES), polyvinylidenefluoride (PVDF), polyamide (PA), and/or polyacrylonitrile (PAN).
In certain embodiments, at least one of the at least two TFF unit operations of the dual stage TFF methods disclosed herein is high performance tangential flow filtration (HPTFF). HPTFF is a two-dimensional unit operation that separates protein solutes on the basis of both size and charge, allowing the separation of protein species that differ by less than 10-fold in size. The primary distinction between TFF and HPTFF unit operations is the use of charged membranes in the latter (in particular, having the same polarity as the species desired to be recovered in the retentate of the unit operation). As known in the art, HPTFF obtains high selectivity by careful modulation of buffer pH and ionic strength to maximize differences in the effective volume of differing charged species in the product/feed stream. This is effected by carefully controlling permeate flux and buffer replacement/diafiltration during operation. The effective volume of a charged protein (*e.g.,* as determined by size exclusion chromatography) accounts for the presence of a diffuse electrical double layer surrounding the protein. Increasing the protein charge, or reducing the solution ionic strength, increases the effective volume of the protein and thus reducing protein transmission through the membrane. Thus, HPTFF effects separations by exploiting differences in both size and charge, with the magnitude of the contributions determined by the properties of the protein, the membrane and the buffer conditions. Optimal separation is typically attained by operating close (1) to the isoelectric point of the species that is to permeate (of filter through) the membrane, and (2) at a relatively low salt concentration to maximize electrostatic interactions. Direct charge effects can also be exploited by selecting filter membranes with favourable charge profiles relative to the species desired to be retained and/or filtered under operating conditions (see, *e.g.,* Zydney and Kuriyel, Methods in Biotechnol. 9(2000), 35-46; Lebreton et al., Biotechnol. Bioeng. 2008(100), 964-974; and U.S. Patent 5,256,294).

As disclosed herein, the dual stage TFF method requires the use of at least two TFF unit operations, comprising (1) a first TFF unit operation that filters a first product stream such that the molecule of interest, *e.g.,* protein, is recovered in the retentate of the TFF unit operation; and (2) a second TFF unit operation that filters a second product stream such that the molecule of interest, *e.g.,* protein, is recovered in the permeate of the TFF unit operation. In certain embodiments, the dual stage TFF methods disclosed herein may be characterized in that (1) the first TFF unit operation comprises an ultrafiltration membrane with a cut-off value that is less than the molecular weight of the molecule of interest (in preferred embodiments at most one-half the molecular weight of the molecule of interest (*i.e.,* a cut-off value of 0.5 times the molecular weight of the molecule of interest or less)) and/or an ultrafiltration membrane that is characterized in that the molecule of interest does not pass through the membrane during the first TFF unit operation; and (2) the second TFF unit operation comprises an ultrafiltration membrane with a cut-off value that is greater than the molecular weight of the molecule of interest (in preferred embodiments at least twice the molecular weight of the molecule of interest (*i.e.,* a cut-off value at least 2 times the molecular weight of the molecule of interest or greater, but less than 1000 kD) and/or an ultrafiltration membrane that is characterized in that the molecule of interest does pass through the membrane during the second TFF unit operation. The ultrafiltration membranes for use according to the methods disclosed herein are not otherwise limited and may be of any material or have any cut-off value(s) provided that they meet the broad exclusion criteria set forth herein (*i.e.,* cut-off value of the membrane in the first TFF unit operation such that the molecule of interest does not pass through the membrane during TFF unit operation; and cut-off value of the membrane in the second TFF unit operation such that the molecule of interest does pass through the membrane during TFF unit operation). Membranes of various materials, cut off ranges, pore sizes and permeability profiles may be prepared by routine methods known in the art (see, *e.g.,* U.S. Patent Application Publication 2010/0190965) and or obtained commercially. For example, Pall GmbH (Dreieich, Germany) offers ultrafiltration membranes having cut-off values of 1, 5, 10, 30, 50, 70, 100, and 300 kD; Satorius AG (Goetting, Germany) offers ultrafiltration membranes having cut-off values of 1, 2, 3, 5, 10, 30, 50, 100 and 300 kD; EMD Millipore (MA, USA) offers ultrafiltration membranes having cut-off values of 1, 3, 5, 10, 30, 100, 300, 500 and 1000 kD; and Novasep (Pompey, France) offers ultrafiltration membranes having cut-off values of 1, 3, 5, 10, 30, 50, 70, 100 and 300 kD.

The dual stage TFF methods disclosed herein are well adapted for use on a commercial scale. The methods may be run in batch or continuous operations, or in a semi-continuous manner, *e.g.,* on a continuous-flow basis of solution containing the desired species, past the at least two TFF unit operations, until an entire large batch has thus been filtered (with one or more optional washing steps interposed between the filtration stages). In this manner, a continuous cycle process can be conducted to give large yields of desired product, in acceptably pure form, over relatively short periods of time.

In certain embodiments, the individual TFF unit operations according to the methods disclosed herein may be combined with diafiltration. Diafiltration is a fractionation process comprising washing smaller molecules through the TFF filter membrane, leaving the larger molecules in the retentate. It is a convenient and efficient technique for concentrating the molecule of interest, and/or removing or exchanging salts, removing detergents, separating free from bound molecules, removing low molecular weight materials, or rapidly changing the ionic or pH environment. Typically, diafiltration is used for buffer exchange wherein the molecule of interest remains in the retentate. Diafiltration may be combined with and/or used in any TFF unit operation of the dual-stage TFF methods disclosed herein. For example, diafiltration may be used in the most upstream TFF unit operation of the dual-stage TFF method, *e.g.,* to replace the feed stream components with a suitable buffer for further processing (see, *e.g.,* Figure 1). Alternately or additionally, diafiltration can be in more than one of the TFF unit operations of the dual-stage TFF method, *e.g.,* during concentration and/or to effect multiple buffer exchanges over the course of processing (see, *e.g.,* Figure 2). Diafiltration processes are well known and routinely used in the art and may be implemented by any suitable method known in the art and/or described herein.

The methods disclosed herein are particularly suited to the elimination of contaminants from feed streams from recombinant protein production processes. Specifically, the methods disclosed are of particular use in the processing of a protein from a cell culture solution and may reduce the common contaminants/impurities HCP and HCDNA such that precipitation of HCP and HCDNA during downstream processing is reduced or eliminated. In particular, the dual stage TFF methods disclosed herein reduce HCP and HCDNA load levels of a product stream (*e.g.,* cell culture solution such as cell culture supernatant) such that the product stream may be adjusted prior to a downstream process, *e.g.,* CEX, without precipitation of HCP and/or HCDNA. In particular embodiments in connection with the processing of a protein from a cell culture solution, the dual stage TFF methods disclosed herein reduce HCP and HCDNA load levels of the product stream such that the solution may be adjusted to a pH of about 5 without precipitation of HCP and/or HCDNA.

In certain embodiments in connection with the separation of a protein from a cell culture solution (*e.g.,* a cell culture supernatant optionally filtered through membrane having a pore size of 0.1µm to 0.45 µm), the dual stage TFF methods disclosed herein may reduce the HCDNA load in the crude feed stream (*i.e.,* initial cell culture solution prior to any unit operation excluding an optional filtration through a filter having a pore size of from 0.1 µm to 0.45 µm), by at least 50%. In other words, the concentration of HCDNA in the solution containing the protein of interest subsequent to and/or resulting from a dual stage TFF method disclosed herein may be reduced by at least 50% relative to the HCDNA concentration in the crude cell culture solution prior to operation of the dual stage TFF method. The methods of the invention have also demonstrated greater efficiency in reduction of HCDNA loads and, in certain embodiments may reduce the concentration of HCDNA in the solution containing the molecule of interest by at least 90% or 95%. Accordingly, the methods disclosed herein may reduce the HCDNA concentration in the solution containing the molecule of interest by at least 50%, at least 55%, at least 60%, at least 65%, at least 70%, at least 75%, at least 80%, at least 85%, at least 90%, or at least 95%.

In certain embodiments in connection with the separation of a protein from a cell culture solution (*e.g.,* a cell culture supernatant optionally filtered through a membrane having a pore size of between 0.1 µm and 0.45 µm), the dual stage TFF methods disclosed herein may reduce the HCP load the crude feed stream (*i.e.,* initial cell culture solution prior to any unit operation excluding an optional filtration through a membrane having a pore size of between 0.1 µm and 0.45 µm), by at least 10%. In other words, the concentration of HCP in the solution containing the protein of interest subsequent to and/or resulting from a dual stage TFF method disclosed herein may be reduced by at least 10%, at least 15%, at least 20% or at least 30%, at least 35%, at least 40%, at least 45% or at least 50% relative to the HCP concentration in the cell culture solution prior to the operation of the dual stage TFF methods of the invention.
In certain embodiments, wherein the molecule of interest is an protein (*e.g.,* antibody) and the feed stream is a cell culture supernatant, the HCP load of the solution containing the molecule of resulting from and/or following implementation of a dual stage TFF method disclosed herein is 450,000 ng per mg of protein or less, and the HCDNA load in the resulting solution is 600,000 pg per mg protein or less.
The antibody according to the current invention is preferably produced by recombinant means. General methods for recombinant production of antibodies are well-known in the state of the art and comprise protein expression in prokaryotic or eukaryotic cells (see for example the following reviews: Makrides, S.C., Protein Expr. Purif. 17, 183-202 (1999); Geisse, S., et al., Protein Expr. Purif. 8 (1996) 271-282; Kaufman, R.J., Mol. Biotechnol. 16 (2000) 151-160; Werner, R.G., Drug Res. 48 (1998) 870-880). The methods according to the current invention are in principal suitable for the production of any antibody. In one embodiment the immunoglobulins produced with the method according to the invention are recombinant immunoglobulins. In other embodiments the immunoglobulins are humanized immunoglobulins, chimeric immunoglobulins, human immunoglobulins, immunoglobulin fragments, or immunoglobulin conjugates. In another aspect of the current invention the antibody is a monoclonal and polyclonal antibody. In a preferred embodiment the antibody is a monoclonal antibody. The antibodies produced by the methods according to the current invention may be therapeutic or diagnostic antibodies. In one embodiment the antibodies are therapeutic antibodies. Examples of therapeutic antibodies include an antibody directed to a tumor antigen (*e.g.* growth factor receptors and growth factors) such as EGFR, HER3, HER4, Ep-CAM, CEA, TRAIL, TRAIL-receptor 1, TRAIL-receptor 2, lymphotoxin-beta receptor, CCR4, CD19, CD20, CD22, CD28, CD33, CD40, CD80, CSF-IR, CTLA-4, fibroblast activation protein (FAP), hepsin, melanoma-associated chondroitin sulfate proteoglycan (MCSP), prostate-specific membrane antigen (PSMA), VEGF receptor 1, VEGF receptor 2, IGF1-R, TSLP-R, PDGF-R, TIE-I, TIE-2, TNF-alpha, TNF like weak inducer of apoptosis (TWEAK), IL-IR, VEGF, EGF, PDGF, HGF and angiopoietin. The antibody according to the present invention may also be alemtuzumab, apolizumab, cetuximab, epratuzumab, galiximab, gemtuzumab, ipilimumab, labetuzumab, panitumumab, rituximab, nimotuzumab, mapatumumab, matuzumab, pertuzumab, or ING-I.

In certain embodiments, the molecule of interest is a protein having a molecular weight of 150 ± 75 kD (*e.g.,* 150 ± 30 kD or 150 ± 15 kD), and the dual stage TFF comprises the use of a first TFF unit operation with an ultrafiltration membrane having a cut-off value of 50 kD; a second TFF unit operation with an ultrafiltration membrane having a cut-off value of 300 kD; and an optional third TFF unit operation with an ultrafiltration membrane having a cut-off of 50 kD. The use of an ultrafiltration membrane having a cut-off value of 50 kD eliminates double-stranded nucleic acids having a size below 240-475 base pairs from the retentate (containing protein of interest); the use of an ultrafiltration membrane having a cut-off value of 300 kD eliminates double-stranded nucleic acid having a size greater than 1450-2900 base pairs acids from the permeate (containing the protein of interest; *i.e.,* double stranded nucleic acids having a size greater than 1450-2900 base pairs are retained within the TFF retentate while the molecule of interest passes through the membrane and may be collected from the permeate). Where the optional third TFF unit operation is downstream of the first and second TFF unit operations, the optional third TFF unit operation may be used to concentrate the protein from the permeate solution resulting from the second TFF unit operation.

For all embodiments disclosed herein, the optional third TFF unit operation may comprise the use of an ultrafiltration membrane identical to or different from that used in the first TFF unit operation; that is, characterized by having a cut-off value less than the molecular weight of the protein of interest and/or characterized in that the protein does not pass through the membrane and may be recovered in the retentate to the third TFF unit operation. In certain embodiments, the third TFF unit operation is characterized as comprising an ultrafiltration membrane having a cut-off value is no more that 0.5 times the molecular weight of the protein of interest is characterized as comprising an ultrafiltration membrane that does not allow the protein of interest to pass through during operation. As with the descriptors "first" and "second" used in connection with a TFF unit operation of the present invention, the descriptor "third" in connection with a TFF unit operation according to the present dual stage TFF methods does not imply any limiting order to the implementation of the individual TFF unit operation in the disclosed method; that is, the third TFF unit operation need not necessarily follow, *i.e.,* be downstream, of the first and second TFF unit operations as disclosed herein. Rather, the third TFF unit operation may be upstream of first TFF unit operation, upstream of the second TFF unit operation, or may be upstream of both the first and the second TFF unit operation. Alternatively or additionally, the third TFF unit operation may be downstream of first TFF unit operation, downstream of the second TFF unit operation, or may be downstream of both the first and the second TFF unit operation. In preferred embodiments, the dual stage TFF method comprises the use of a first TFF unit operation, a downstream second TFF unit operation and a third TFF unit operation that is downstream of both the first and second unit operation, wherein the third TFF unit operation comprises the use of an ultrafiltration membrane that may or may not be identical to that used in the first TFF unit operation and/or that is no more than 0.5 times the molecular weight of the protein of interest.

In certain embodiments, the molecule of interest is a protein having a molecular weight of 150 + 75 kD, and the dual stage TFF comprises the use of a first TFF unit operation with an ultrafiltration membrane having a cut-off value of 50 kD, a downstream second TFF unit operation with an ultrafiltration membrane having a cut-off value of 300 kD, and a third TFF unit operation with an ultrafiltration membrane having a cut-off of 50 kD that is downstream of both the first and second TFF unit operation. The first, second, and/or third TFF unit operations may be combined with diafiltration. The first, second, and/or third TFF unit operations may be implemented in batch and directly following one another or may be implemented sequentially as a continuous flow system. The dual stage TFF methods disclosed herein also encompass the use of one or more unit operations interspersed between the first, second, and/or third TFF unit operations according to the invention. One or more of the first, second, and/or third TFF unit operations may be operated in parallel with the other TFF unit operations of the invention or may be operated in parallel with one or more additional unit operations.

The dual stage TFF methods disclosed herein in conjunction with downstream CEX offer distinct advantages for protein processing over corresponding processes known in the art, in particular, providing advantages for further downstream processing/purification of proteins. Specifically, in comparison with processes comprising a single stage TFF, the downstream processing (*e.g.,* CEX) of feed and/or product streams containing the protein of interest that were treated according to the methods of the invention offered distinct processing and economic advantages. For example, processing of a cell culture supernatant using dual stage TFF according to the present methods surprisingly eliminated the need for any precipitation and precipitate filtration processes prior to CEX. In contrast, the process comprising a single stage TFF method required the insertion of additional precipitation/precipitate filtration step to remove HCDNA and HCP contaminants from the product stream prior to CEX. Accordingly, the methods presently disclosed herein offer the advantages of saving direct and indirect time and money during the downstream purification process.

The requirement for precipitation/precipitate filtration in non-affinity processes of the prior art results from the inability of single stage TFF processing according to known methods to sufficiently remove contaminants (*e.g.,* HCP and/or HCDNA) from the sample stream prior to downstream chromatography processing. For example, non-affinity protein processing/purifications according to methods known in the art typically use AEX and/or CEX for further refining/purifying a product stream. However, the contamination levels resulting from a single stage TFF typically preclude the use of either AEX or CEX immediately downstream, due to overloading of the column capacity (see, for example, the Example sections herein). Further, failure to sufficiently purify the contaminants from the sample stream also may lead to precipitation on the pH/buffer adjustment necessary prior to many chromatography purification processes, *e.g.,* CEX processing. Accordingly, the contaminant levels themselves and/or their precipitation during standard processing methods can negatively impact chromatography processes used in place of affinity purification. However, the necessary removal of contaminants and, in particular, the resulting precipitate adds significant time and costs to the purification process (*e.g.,* refrigeration costs, as industrial precipitations are usually conducted at 4 to 8 °C overnight). Precipitation processes also suffer from the disadvantage that the recovery of the product may be poor, depending on the nature and volume of the precipitate. For example, it is typically not suitable to apply both the precipitate and the supernatant to a depth filter, due to induction of filter blocking; therefore, the soluble product that is present in the precipitate fraction is usually lost.

In contrast, the methods of the present invention (*i.e.,* comprising dual stage TFF as disclosed herein) surprisingly reduce product stream contaminants to concentrations exhibiting minimal or no impact on downstream processes, *e.g.,* eliminating the precipitation/precipitate filtration processes normally required on pH adjustment. For example, using the single stage TFF processing of a cell culture supernatant known in the art, HCDNA may be reduced by a mere 17 to 24%; in contrast, the dual stage TFF methods of the present invention may reduce HCDNA levels in a cell culture supernatant by at least 85% to 92%, and potentially greater. Similarly, using the single stage TFF processing known in the art, HCP may be reduced in a cell culture supernatant by only 0 to 28%; in contrast, the dual stage TFF methods of the present invention may reduce HCP levels of a cell culture supernatant by at least 17 to 46% or greater.

With respect to the individual processes of the dual stage TFF methods disclosed herein, the second TFF unit operation may provide the majority of contaminant purification from solutions containing the molecule of interest, *e.g.,* protein, in particular, where the feed stream to the process is a cell culture solution (*i.e.,* a cell culture supernatant). For example, operation of the second TFF unit operation, *i.e.,* characterized by the molecule of interest being recovered in the permeate, on a cell culture supernatant feed stream may eliminate at least 86% of the HCDNA load and at least 22% of the HCP load from the resulting solution containing the protein of interest. The second TFF unit operation may also eliminate at least 50%, at least 55%, at least 60%, at least 65%, at least 70%, at least 75%, at least 80% at least 85% or at least 90% of the HCDNA load in the solution containing the molecule of interest. The second TFF unit operation may also eliminate at least 5%, at least 10%, at least 15%, at least 20%, at least 25% or at least 30% of the HCP load in the solution containing the molecule of interest.

As disclosed herein, the significant reduction in contaminant levels according to the dual stage TFF methods disclosed herein has significant effects with respect to both downstream and upstream processes. For example, the robust removal of contaminants using the dual stage TFF methods disclosed herein renders the methods effective over a wide range of feed stream quality, *e.g.,* effective over a wide range of feed stream contaminant levels/concentrations. Thus, the methods disclosed herein may be implemented without regard to feed stream quality (*e.g.,* quality of the conditioned, clarified cell culture supernatant) and, may eliminate dependence on upstream processing to satisfy minimum quality requirements. Similarly, the significant elimination of contaminants may also remove considerations regarding downstream processing, such as ion exchange column capacity, allowing an increased flow and improved processing time. The dual stage TFF processing is also less time consuming compared to current methods known in the art, *e.g.,* comprising precipitation and precipitate filtration steps.

The individual TFF unit operations forming the dual stage TFF method of the present disclosure (*i.e.,* comprising a first and a second TFF unit operation and, optionally, one or more third TFF unit operations), may be joined in combination to provide a continuous flow process or may be individually performed as separate batch operations. The individual TFF unit operations of the methods disclosed herein may also be joined in parallel as known in the art, *e.g.,* such that the product stream of one operation feeds another operation, which product stream is then recirculated to the original operation. The use of the individual unit operations in parallel may also mitigate costs associated with increased storage capacities and processing times.

The presently disclosed dual stage TFF methods may be directly upscaled as recognized in the art. This allows it use in the in any process where precipitation free downstream processes are desired. For example, the dual stage TFF may be implemented prior to any chromatography step in order to increase the column life time. The dramatic reduction of HCP and HCDNA load may result in increased column life through both direct and indirect means. In a direct manner, the methods of the invention reduce the amount of impurities that contact the adsorber, preventing filter blockage/fouling. Indirectly, the contaminant reduction may allow for a reduction in the harshness of the cleaning-in-place conditions required for column regeneration. The methods of the present invention may find particular use for the purification of antibodies for diagnostic purposes (non-Pharma).

### EXAMPLES

### Example 1:

### Processing Of Protein From A Solution Using Single Stage TFF

The present example relates to the isolation and/or purification of an antibody from a solution containing the antibody using non-affinity purification methods, in particular, anion exchange chromatography ("AEX") and cation exchange chromatography ("CEX"). The initial solution containing the antibody is a feed stream of clarified conditioned cell culture media. The feed stream is preconditioned using a single stage of tangential flow filtration ("TFF") having a 50 kD cut-off. The present example relates to a common practice, routinely used in the art during non-affinity isolation of antibody from typical feed streams (see, *e.g*., Gottschalk 2009; Alahari et al., BioPharm Int. March 2, 2009 (Supp.); Wang et al., BioPharm. Int. October 2, 2009). In particular, the antibody is first concentrated (and partially purified as disclosed herein) using a single stage of TFF using an ultrafiltration membrane having a cut-off value below the molecular weight of the antibody. The TFF process is followed by a buffer exchange/diafiltration prior to the chromatography processes. Although irrelevant for the particular methods disclosed and exemplified herein, the specific antibody used for this example was an anti-colony stimulating factor 1 receptor antibody (anti-CSFRl; see, WO 2011/131407).

### Methods:

### Single Stage Tangential Flow Filtration And Diafiltration (Product In Retentate)

The single stage tangential flow filtration was effected using a Satorius Sartocon Slice unit with Tandem Pump Model 1082 (Satorius Stedim Biotech S.A., Aubagne, France) and a Sartocon Slice Cassette PESU 50 kD (200 cm² filtration area, product # 3081465002 E-SG; Satorius Stedim). In the single stage of TFF, 945 ml of clarified cell culture supernatant (*i.e.,* the feed stream) containing the antibody of interest at a concentration of 2.9 mg/ml was concentrated to 136 ml.

Buffer exchange was subsequently conducted by diafiltration against a 10-fold volume of 25 mM Tris/HCl, 50 mM NaCl, pH 7.5. The TFF/diafiltration conditions were selected according to standard practices in the art. In particular, cut-off value of the single stage TFF, 50 kD, is exemplary of the 10 to 50 kD cut-off that routinely selected so as to concentrate the product (*i.e.,* the immunoglobulin) while filtering low molecular weight host-cell proteins, DNA and product fragments from the product stream.

### Anion Exchange Chromatography

The product stream from the TFF/buffer exchange step (*i.e.,* the retentate) was subject to anion exchange chromatography ("AEX") according to standard methods known in the art. The chromatography conditions were as follows:

| | |
|---|---|
| Exchange material: | Q-Sepharose FF (GE Healthcare, Freiburg, Germany) |
| Column: | 8 mm internal diameter, 100 mm length, 5.03 ml volume |
| Flow rate: | 150 cm/h (1.25 ml/min)* |
| Equilibration solution: | 25 mM Tris/HCl, 50 mM NaCl, pH 7.5 |
| Loading: | 80 g protein/l chromatography material |
| Wash solution: | 25 mM Tris/HCl, 50 mM NaCl, pH 7.5 |
| Elution method: | isocratic |

| | |
|---|---|
| (*) The flow rate was reduced due to an increase of back-pressure observed during sample loading and elution. | |

The antibody-containing sample (product feed or product stream) was loaded on the chromatography column as indicated above. After loading, the column was washed with wash solution and the antibody recovered using an isocratic elution method (flow-through mode).

### pH adjustment to 5.0

As indicated above, the antibody eluted from the AEX column was in a buffer having a pH of 7.5. In order to conduct the subsequent CEX, the antibody-containing eluate was pH adjusted to a pH of 5.0 by the addition of 1 M citric acid.

As has often been reported in the art, pH adjustment at this corresponding stage in processing resulted in the precipitation of feed contaminants, e.g., host cell proteins ("HCP") and host cell DNA ("HCDNA"; see, *e.g.,* Gottschalk 2006; Anakumari et al., BioPharm Int. Feb. 2007 (Supp); Arunakumari et al., BioPharm. Int. Mar. 2, 2009 (Supp); Arunakumari, Bioprocess Int. Feb. 2009; Jue et al., BioPharm Int. Mar. 2, 2008 (Supp); Jue et al.., BioPharm Int. Oct. 2, 2009). Therefore, prior to CEX, the sample had to be clarified by centrifugation and filtration.

### Cation Exchange Chromatography: Bind and Elute Mode

Subsequent to pH adjustment and filtration, the antibody was isolated from the product stream using CEX. The chromatography conditions were as follows.

| | |
|---|---|
| Exchange material: | Poros 50HS (Life Technologies Co., Carlsbad, CA, USA) |
| Column: | 8 mm internal diameter, 100 mm length, 5.03 ml volume |
| Flow rate: | 150 cm/h (= 1.25 ml/min) |
| Equilibration solution: | 10 mM sodium citrate, pH 5.0 |
| Loading: | 40 g protein/l chromatography material |
| Wash solution: | 10 mM sodium citrate, pH 5.0 |
| Elution solution: | 10 mM sodium citrate, 750 mM NaCl, pH 5.0 |
| Elution method: | linear gradient from 0 % (v/v) to 100 % (v/v) elution solution in 22.5 column volumes |

After the loading the CEX column as indicated above, the column was washed with wash solution. The antibody in monomeric form was recovered with a linear gradient elution method, whereby the pH value was held constant and the conductivity was varied (increased) by the addition of sodium chloride.

### Antibody and Contaminant Determination

For initial and final product streams, as well as at various intermediate stages of the purification process (typically after each unit operation), the concentrations of both the antibody and of the contaminants, host cell proteins ("HCP") and host cell DNA ("HCDNA"), were determined. The antibody concentration for the clarified cell culture supernatant (initial feed stream) and in all intermediate product streams was determined by Protein A-HPLC (Protein A affinity high performance liquid chromatography; employed column: PA ImmunoDetection® Sensor Cartridge for Perfusion Immunoassay™ Technology from Applied Biosystems). The antibody concentration of the in the pooled eluate from the CEX was determined by UV 280 nm adsorption.

The proportion of monomeric antibody was determined by SE-HPLC (size exclusion high performance liquid chromatography; employed column: TSK-GEL G3000SWXL; 7.8 mm ID x 30.0 cm L, 5 µm from Tosoh Bioscience).

The residual host cell protein (HCP) was determined by a polyclonal sandwich type enzyme-linked immunosorbent assay (ELISA). Briefly, microtiter plates pre-coated with streptavidin were coated with antibodies against Chinese hamster ovary (CHO) HCP labeled with biotin via biotin-streptavidin interaction and incubated with "product antibody" solution. Bound HCP was detected by anti-CHO HCP antibodies conjugated with digoxigenin combined with anti-digoxigenin-antibodies conjugated with horse radish peroxidase. The bound CHO HCP was visualized by adding the substrate 2,2'-azino-bis(3-ethylbenzthiazoline-6-sulphonic acid) (ABTS). In parallel to the measurements of "product antibody" solutions, a standard curve was monitored.

A q-PCR based assay was used for the quantitative determination of CHO DNA. Samples (diluted with assay buffer if necessary) were lysed under highly denaturing conditions to ensure isolation of intact DNA. The isolated DNA bound to a silica-gel-based membrane and was separated from the remaining buffer and sample matrix by centrifugation. DNA was eluted in elution buffer. After heat denaturation of the DNA, forward and reverse primer bound selectively to the target sequence. Between the two primers a sequence specific CHO DNA probe hybridized with the target DNA.

The probe was labeled with a fluorescent reporter dye at the 5'end and with a quencher dye at the 3'end. As long as the probe remains intact, fluorescence is quenched due to relative proximity of the quencher dye to the reporter dye. During amplification, the Taq-Polymerase hydrolyses the probe bound to the target sequence due to its 5'-->3' exonuclease activity. The reporter dye is released and the increase of fluorescence intensity is directly proportional to the amount of PCR product. The amount of DNA in the sample was quantified by an external standard curve.

For the final result the measured CHO DNA concentration was multiplied by the dilution factor of the sample, if applicable, and divided by the protein concentration.

### Results

The concentration of antibody, the proportion of antibody monomer and contaminant levels for the initial feed stream, the intermediate product streams and the final eluate are provided in Table 1. Step yield provides the percentage of antibody retained for that particular unit operation

**Table 1**

| **Sample** | **Antibody concentration [mg/ml]** | **Step yield (monomeric antibody) [%]** | **SE-HPLC monomer [%]** | **HCP level [ng/mg]** | **HCDNA level [pg/mg]** |
|---|---|---|---|---|---|
| Clarified cell culture supernatant (fresh) | 2.90 | 100 | n.d. | 245635 | 6982759 |
| TFF 50 kD cut-off (retentate) | 15.23 | 77 | 68.80 | 176162 | 5300722 |
| AEX elution pool, pH7.5 | 9.2 | 90 | 72.9 | 62467 | 5546739 |
| pH adaptation (pH 7.5 to 5.0) AEX elution pool, pH 5.0 | 9.2 | 24.6 | 73.41 | 24990 | 4272 |
| CEX elution pool | 6.45 | 73 | 86,10 | 3049 | 74 |
| ***Overall yield*** | | ***12%*** | | | |

| | | | | | |
|---|---|---|---|---|---|
| n.d. (not determined) | | | | | |

As reported above, the initial feed stream was 945 ml of clarified cell culture supernatant having an antibody concentration of 2.9 g/l. The HCP load was 245635 ng/mg, the HCDNA load was 6982759 pg/mg.

Following the single stage TFF with 50 kD cut-off and buffer exchange/diafiltration, the calculated step yield for monomeric antibody was approximately 77% (*i.e.,* 77% of the monomeric antibody in the applied feed was recovered from the unit operation). The "monomer peak" of the retentate detected by SE-HPLC was 69%. The single stage TFF reduced the HCP load in the product stream by 28% and the HCDNA load by 24%.

AEX subsequent to TFF/diafiltration was operated in flow through mode. Column loading was 80 g/l. During the loading of the sample onto the AEX column, a dramatic increase in column back-pressure was observed. However, the loading process was not stopped but was continued with a reduced volumetric flow. It is believed that the increase in back-pressure resulted from the high HCDNA concentration of the feed product stream (5300722 pg/mg), which overloaded the capacity of the AEX column. Based on the HCDNA ELISA data, it was calculated that the column was confronted with 0.42 µg/ml HCDNA (5300722 pg/mg HCDNA x 80 mg/ml loading). Prevention of overloading faced with similar HCDNA concentrations would have required a significantly larger column.

The calculated step yield for the AEX (monomeric antibody) was 90% (*i.e.,* 90% of the monomeric antibody of the applied feed solution (from the single stage TFF/diafiltration) was recovered after this unit operation). The "monomer peak" detected by SE-HPLC was 73%, slightly higher compared with the feed sample (69%). The buffer exchange/AEX unit operation reduced HCP load of the product stream by 65%, while the HCDNA load was slightly increased by 5%. The HCDNA data illustrate that the AEX step does not significantly contribute to the purification process with respect to HCDNA, at least in this experiment. The lack of purification with respect to HCDNA in this experiment is believed due to column overloading, the prevention of which could potentially be remedied with the use of a significantly larger column.

Prior to the CEX step, the pH of the product stream was adjusted to 5.0. Precipitation was observed in the sample at this stage. It was assumed that only impurities precipitated because the antibody concentration of the product stream did not change relative to the value before pH adjustment. Precipitates were removed by filtration. However, the pH adjustment/filtration step was associated with a low product yield of only 25%. This is attributed to volumetric loss of the sample during filtration at a small scale. Extensive impurity precipitation events will most likely lead to a decrease in product recovery (as observed here), due to volumetric losses for a corresponding precipitation and filtration step. However, it can be expected that the corresponding volumetric losses at a large scale process would be reduced compared to the small scale data presented here.

Following pH adjustment and filtration, the "monomer peak" of the product detected by SE-HPLC is 73%, which is identical compared to the feed sample. The pH adjustment/filtration reduced the HCP load of the product stream by 60% and reduced the HCDNA load by 99.9%.

The product stream was subsequently loaded on CEX column operated in bind-and-elute mode. The calculated step yield (monomeric antibody) was 73%. For the product, the "monomer peak" detected by SE-HPLC was 86%, which was significantly higher compared to that in the feed sample (73%). CEX reduced the HCP and HCDNA load of the product stream by 88% and 98%, respectively. The removal of antibody aggregates and fragments, as well as the dramatic reduction of HCP and HCDNA, demonstrate that CEX efficiently removes the remaining impurities at a column loading of 40 g/l.

### Example 2:

### Processing Of Protein From a Solution Using Dual Stage TFF

The present example relates to the isolation and/or purification of an antibody from a solution containing the antibody using a non-affinity purification method, in particular, AEX and CEX. The initial solution is a feed stream of clarified, conditioned cell culture media. This example is identical to Example 1 with the exception that the feed stream was preconditioned using dual stage tangential flow filtration. In this experiment, the first TFF unit operation of the dual stage TFF was identical to the single TFF stage used in Example 1, *i.e.,* conducted with an ultrafiltration membrane having a 50 kD cut-off value and followed by a buffer exchange/diafiltration. However, unlike example 1, the present study demonstrates the addition of a second TFF unit operation conducted with a column having a 300 kD cut-off, providing dual stage TFF. This set up may be seen in Figures 1 or 2. The exemplified study further added a third TFF/concentration unit operation subsequent to the 300 kD TFF, conducted with a column having a 50 kD cut-off. A schematic of the dual stage TFF used in this example is provided in Figure 3. The second and third TFF unit operations (optionally combined with diafiltration) may be combined in parallel as shown in the schematic in Figure 4. Following the dual stage TFF processing (TFF 50 kD, TFF 300 kD, TFF 50 kD), AEX and CEX were conducted as in Example 1 (with the exception of a centrifugation/filtration step as explained below). This study was conducted with the anti-CSFR1 antibody used in Example 1.

### Methods:

### First Stage Tangential Flow Filtration And Diafiltration (Product In Retentate)

The first stage TFF and diafiltration were effected as described for the single stage TFF reported in Example 1. In the first stage, 945 ml of clarified cell culture supernatant containing the antibody of interest at a concentration of 2.9 mg/ml was concentrated to 136 ml.

Subsequently, buffer exchange was conducted by diafiltration against a 10-fold volume of 25 mM Tris/HCl, 50 mM NaCl, pH 7.5.

### Second Stage Tangential Flow Filtration (Product In Permeate)

The second stage TFF was conducted similar to the TFF of the first stage, but using a Sartocon

Slice Cassette PESU 300 kD (200 cm² filtration area, product # 3081467902E-SG; Satorius Stedim). During this second stage TFF, the product (antibody) passes through the filter and is found in the permeate (accordingly, the permeate of this second stage TFF forms the product stream). During this second stage TFF, 108 ml of sample containing the antibody at a concentration of 15.2 mg/ml (*i.e.,* the retentate from the first stage TFF) was diafiltered on the second column against a 15-fold buffer volume (25 mM Tris/HCl, 50 mM NaCl, pH 7.5).

### Concentration

The second stage TFF was designed such that the product is in the permeate and results in a dramatic increase in the volume of the sample containing the product (*i.e.,* a dramatic increase in the volume of the product stream). Accordingly, a third TFF using a column with a 50 kD cut-off was used to concentrate the product and reduce the volume of the product stream. This third TFF stage used a column with a 50 kD cut-off identical to that in the first stage TFF described above and in Example 1. In this third stage, 1627 ml of the product stream at a concentration of 1.1 mg/ml (*i.e.,* the permeate from the second stage TFF) was concentrated into a volume of 143 ml.

### Anion Exchange Chromatography

AEX following the dual stage TFF processing step proceeded as in Example 1. The chromatography conditions were as follows:

| | |
|---|---|
| Exchange material: | Q-Sepharose FF (GE Healthcare, Freiburg, Germany) |
| Column: | 8 mm internal diameter, 100 mm length, 5.03 ml volume |
| Flow rate: | 150 cm/h (1.25 ml/min) |
| Equilibration solution: | 25 mM Tris/HCl, 50 mM NaCl, pH 7.5 |
| Loading: | 86 g protein/l chromatography material |
| Wash solution: | 25 mM Tris/HCl, 50 mM NaCl, pH 7.5 |
| Elution method: | isocratic |

The product feed was loaded on the chromatography column as indicated above. After loading, the column was washed with wash solution and the antibody recovered using an isocratic elution method (flow-through mode). Note that in contrast to Example 1, no dramatic increase in back-pressure during AEX was observed, and no reduction in flow rate was necessary.

### pH adjustment to 5.0

As in Example 1, prior to CEX, the sample eluted from the AEX column was pH adjusted to a pH of 5.0 by the addition of 1 M citric acid. However, in contrast to Example 1, no precipitation was observed at this stage and, thus, no centrifugation/filtration of the product stream was necessary. Rather, the product stream was loaded directly on the CEX column.

### Cation Exchange Chromatography: Bind and Elute Mode

Subsequent to pH adjustment, the antibody was isolated from the product stream using CEX as described in Example 1. The chromatography conditions were as follows.

| | |
|---|---|
| Exchange material: | Poros 50HS (Life Technologies Co., Carlsbad, CA, USA) |
| Column: | 8 mm internal diameter, 100 mm length, 5.03 ml volume |
| Flow rate: | 150 cm/h (= 1.25 ml/min) |
| Equilibration solution: | 10 mM sodium citrate, pH 5.0 |
| Loading: | 20 g protein/l chromatography material |
| Wash solution: | 10 mM sodium citrate, pH 5.0 |
| Elution solution: | 10 mM sodium citrate, 750 mM NaCl, pH 5.0 |
| Elution method: | linear gradient from 0 % (v/v) to 100 % (v/v) elution solution in 22.5 column volumes |

After the loading the CEX column as indicated above, the column was washed with wash solution. The antibody in monomeric form was recovered with a linear gradient elution method, whereby the pH value was held constant and the conductivity was varied (increased) by the addition of sodium chloride.

### Antibody and Contaminant Determination

The antibody concentration and the concentration of the contaminants, host cell proteins ("HCP") and host cell DNA ("HCDNA"), was determined at various stages of the experiment according to the methods described in Example 1.

### Results

The concentration of antibody, the proportion of antibody monomer and contaminant levels for the initial feed stream, the intermediate product streams and the final eluate are provided in Table 2. Step yield provides the percentage of antibody retained for that particular unit operation.

**Table 2**

| **Sample** | **Antibody concentration [mg/ml]** | **Step yield (monomeric antibody) [%]** | **SE-HPLC monomer [%]** | **HCP level [ng/mg]** | **HCDNA level [pg/mg]** |
|---|---|---|---|---|---|
| Clarified cell culture supernatant (fresh) | 2.90 | 100 | n.d. | 245635 | 6982759 |
| TFF 50 kD cut-off (retentate) | 15.23 | 77 | 68.80 | 176162 | 5300722 |
| TFF 300 kD cut-off (permeate) | 1.13 | 100 (> 100) | 71.96 | 138600 | 330088 |
| TFF 50 kD cut-off (retentate) | 12.0 | 93 | 72.44 | 132169 | 588750 |
| AEX elution pool, pH7.5 | 8.6 | 92 | 75.25 | 19853 | 61012 |
| pH adaptation (pH 7.5 to 5.0) AEX elution pool, pH 5.0 | n.d | n.d | n.d | 16478 | 37558 |
| CEX elution pool | 6.18 | 90 | 95.86 | 1449 | 223 |
| ***Overall yield*** | | ***59%*** | | | |

| | | | | | |
|---|---|---|---|---|---|
| n.d. (not determined) | | | | | |

As reported above, the initial feed stream was 945 ml of clarified cell culture supernatant having an antibody concentration of 2.9 g/l. The HCP load was 245635 ng/mg, the HCDNA load was 6982759 pg/mg.

Following the first stage TFF with 50 kD cut-off and buffer exchange/diafiltration, the calculated step yield for monomeric antibody was approximately 77 %. The "monomer peak" of the product stream (retentate) detected by SE-HPLC was 69%. The single stage TFF reduced the HCP load in the product stream by 28% and the HCDNA load by 24%. This is the identical unit operation as the single stage TFF reported in Example 1.

Following the second stage TFF with a 300 kD cut off, the calculated step yield for monomeric antibody was 112%. The "monomer peak" detected by SE-HPLC was 72%, which is slightly higher than that of the applied feed (69%). The second stage TFF reduced the HCP load and HCDNA load of the product stream by 22% and 94%, respectively.

For the subsequent TFF/concentration step using a column with a 50 kD cut-off, the calculated step yield (monomeric antibody) was 93%. For the eluted sample, the "monomer peak" detected by SE-HPLC was 72%, which was identical to the applied feed sample. The TFF/concentration stage reduced the HCP load of the product stream by 5%, while the HCDNA load increased by 78% (which is attributed to assay variation).

AEX subsequent to dual stage TFF peconditioning was operated in flow through mode. Column loading was 86 g/l. The calculated step yield for the AEX (monomeric antibody) was 92%. The "monomer peak" detected by SE-HPLC was 75%, slightly higher compared to the feed sample (72%). The AEX unit operation reduced HCP load of the product stream by 85%, while the HCDNA load was reduced by 89%. In contrast to AEX using a single stage TFF preconditioning as in Example 1, the HCP and HCDNA data indicate that AEX subsequent to the dual stage TFF preconditioning contributed significantly to the purification process, even though it is operated in flow-through mode at comparably high column loading.

Prior to the CEX step, the pH of the product stream was adjusted to 5.0. In contrast to the single stage TFF of Example 1, no precipitation was observed in the sample at this stage and the sample/product stream was loaded directly onto the CEX column.

The CEX column was operated in bind-and-elute mode. The calculated step yield (monomeric antibody) was 90%. For the product, the "monomer peak" detected by SE-HPLC was 96%, which was much higher compared to that in the feed sample (72%). CEX reduced the HCP and HCDNA load of the product stream by 73% and 99%, respectively. The removal of antibody aggregates and fragments, as well as the dramatic reduction of HCP and HCDNA, demonstrate that CEX efficiently removes the remaining impurities at a column loading of 20 g/l.

### Example 3:

### Processing Of Protein From A Solution Using Single Stage TFF

Example 1 was repeated using a feed stream containing a different protein of interest, in particular, an anti-angiopoietin 2 (Ang2)/VEGF antibody (see, *e.g.,* U.S. Patent Application publication 2010/0111967). Only the deviations from the methods of Example 1 are reported herein.

### Methods:

### Single Tangential Flow Filtration And Diafiltration (Product In Retentate)

In the single stage TFF, 950 ml of clarified cell culture supernatant containing the anti-Ang2/VEGF antibody at a concentration of 2.7 mg/ml was concentrated to 134 ml. Buffer exchange was also subsequently effected identically with Example 1.

### Anion Exchange Chromatography

AEX proceeded according to Example 1 with the exception that the loading of the column was 19 g protein/l column material. In contrast to the AEX process reported in Example 1, no increased back-pressure was observed at this stage of the anti-Ang2/VEGF antibody processing; the 150 cm/h flow rate was maintained during the entire unit operation. This is likely due to the dramatically lower concentration of HCDNA in the initial anti-Ang2/VEGF antibody feed stream as compared to the initial anti-CSFR1 feed stream in Example 1 (approximately 80% less; compare Tables 1 and 3). This results in a lower concentration of HCDNA for all unit operations prior to precipitation and filtration.

### pH adjustment to 5.0

Despite the lower concentration of HCDNA throughout the present process as compared to that in Example 1, precipitation was also observed for the anti-Ang2/VEGF product stream during pH adjustment to 5.0. Again, it was assumed that only contaminants precipitated as the concentration of antibody in the product stream was not altered. As in Example 1, the sample was clarified by centrifugation and filtration prior to CEX.

### Cation Exchange Chromatography: Bind and Elute Mode

CEX of the anti-Ang2/VEGF sample was performed as in Example 1 with the exception of decreasing the column loading to 13 g protein/l chromatography material.

### Antibody and Contaminant Determination

The antibody concentration and the concentration of the contaminants HCP and HCDNA were determined throughout the experiment as described in Example 1.

### Results

The concentration of antibody, the proportion of antibody monomer and contaminant levels for the feed stream, the intermediate product streams and the final eluate are provided in Table 3.

**Table 3**

| **Sample** | **Antibody concentration [mg/ml]** | **Step yield (monomeric antibody) [%]** | **SE-HPLC monomer [%]** | **HCP level [ng/mg]** | **HCDNA level [pg/mg]** |
|---|---|---|---|---|---|
| Clarified cell culture supernatant (fresh) | 2.66 | 100 | n.d. | 420531 | 14443609 |
| TFF 50 kD cut-off (retentate) | 11.9 | 86 | 65.38 | 444247 | 1194958 |
| AEX elution pool, pH7.5 | 6.3 | 79 | 78.34 | 167790 | 1225397 |
| pH adaptation (pH 7.5 to 5.0) AEX elution pool, pH 5.0 | 6.3 | n.d. | n.d. | n.d. | n.d. |
| CEX elution pool | 3.14 | 99 | 93.69 | 16308 | 65 |
| ***Overall yield*** | | ***67%*** | | | |

| | | | | | |
|---|---|---|---|---|---|
| n.d. (not determined) | | | | | |

As reported above, the initial feed stream was 950 ml of clarified cell culture supernatant having an antibody concentration of 2.7 g/l. The HCP load was 420531 ng/mg, the HCDNA load was 1443609 pg/mg.

Following the single stage TFF with 50 kD cut-off and buffer exchange/diafiltration, the calculated step yield for monomeric antibody was approximately 86%. The "monomer peak" of the retentate detected by SE-HPLC was 65%. The single stage TFF increased the HCP load in the product stream by about 6% and reduced the HCDNA load by 17%.

AEX subsequent to TFF/diafiltration was operated in flow through mode. Column loading was 79 g/l. The calculated step yield for the AEX (monomeric antibody) was 79%. The "monomer peak" detected by SE-HPLC was 78%, slightly higher compared with the feed sample (65%). The buffer exchange/AEX unit operation reduced HCP load of the product stream by 62%, while the HCDNA load was slightly increased by 3%. Although HCP removal was achieved to a certain extent, the present data confirm the results of Example 1, namely that the AEX step subsequent to a single stage of TFF processing does not significantly contribute to the purification process for the antibody. Again, consistent with Example 1, it is believed that the AEX processing did not significantly contribute to a reduction in HCDNA concentration due to overloading of the column. Prevention of overloading may perhaps be remedied by the use of a significantly larger, and more expensive, column.

Prior to the CEX step, the pH of the sample was adjusted to 5.0. Precipitation was again observed in the present product stream, despite the sample having a significantly reduced concentration of HCP and HCDNA compared to the corresponding sample of Example 1. Again, it was assumed that only impurities were precipitated because the antibody concentration of the sample did not change relative to the value before pH adjustment. Precipitates were removed by filtration. Neither antibody monomer nor contaminant analysis were conducted following pH adjustment/precipitation/filtration of the sample.

The sample was subsequently loaded on CEX column operated in bind-and-elute mode. The calculated step yield (monomeric antibody) was 99%. For the product, the "monomer peak" detected by SE-HPLC was 94%. Because the intermediate product stream was not tested for contaminant concentration as reported above, the step contribution of CEX to contaminant reduction could not be evaluated. However, the final HCP load was 16308 ng/mg and the final HCDNA load was 65 pg/ml.

### Example 4:

### Processing Of Protein From A Solution Using Dual Stage TFF

The present example repeats Example 2 using the feed stream of Example 3, *i.e.,* containing an anti-Ang2/VEGF antibody. The methods of Example 2 are not again detailed and only deviations from the methods reported herein.

### Methods:

### First Stage Tangential Flow Filtration And Diafiltration (Product In Retentate)

In the first stage TFF, 950 ml of clarified cell culture supernatant containing the antibody of interest at a concentration of 2.7 mg/ml was concentrated to 134 ml. Buffer exchange proceeded as in Example 2.

### Second Stage Tangential Flow Filtration (Product In Permeate)

In the second stage TFF, 98 ml of product stream sample containing the antibody at a concentration of 11.9 mg/ml (*i.e.,* the retentate from the first stage TFF) were diafiltered against an 18-fold buffer volume.

### Concentration

In the third stage TFF/concentration, 1524 ml of the product stream containing 1.0 mg/ml of antibody was concentrated into a volume of 157 ml.

### Anion Exchange Chromatography

AEX proceeded according to the methods of Example 2, with the exception that the loading was 80 g protein/l chromatography material.

### pH adjustment to 5.0

pH adjustment was conducted as detailed in Example 2.

### Cation Exchange Chromatography: Bind and Elute Mode

CEX was conducted as detailed in Example 2.

### Antibody and Contaminant Determination

The antibody concentration and the concentration of the contaminants, host cell proteins ("HCP") and host cell DNA ("HCDNA"), was determined throughout the experiment according to the methods described in Example 1.

### Results

The concentration of antibody, the proportion of antibody monomer and contaminant levels for the initial feed stream, the intermediate product streams and the final eluate are provided in Table 4.

**Table 4**

| **Sample** | **Antibody concentration [mg/ml]** | **Step yield (monomeric antibody) [%]** | **SE-HPLC monomer [%]** | **HCP level [ng/mg]** | **HCDNA level [pg/mg]** |
|---|---|---|---|---|---|
| Clarified cell culture supernatant (fresh) | 2.66 | 100 | n.d. | 420531 | 1443609 |
| TFF 50 kD cut-off (retentate) | 11.9 | 86 | 65.38 | 444247 | 1194958 |
| TFF 300 kD cut-off (permeate) | 1.0 | 100 | 75.08 | 315945 | 170000 |
| TFF 50 kD cut-off (retentate) | 7.2 | 75 | 76.02 | 347662 | 165000 |
| AEX elution pool, pH7.5 | 3.6 | 90 | 80.07 | 124815 | 17139 |
| pH adaptation (pH 7.5 to 5.0) AEX elution pool, pH 5.0 | 3.6 | 100 | n.d | n.d | n.d |
| CEX elution pool | 6.02 | 100 | 94.80 | 8878 | 29 |
| ***Overall yield*** | | ***58%*** | | | |

| | | | | | |
|---|---|---|---|---|---|
| n.d. (not determined) | | | | | |

As reported above, the initial feed stream was 950 ml of clarified cell culture supernatant having an antibody concentration of 2.7 g/l. The HCP load was 420531 ng/mg, the HCDNA load was 1443609 pg/mg.

Following the first stage TFF with 50 kD cut-off and buffer exchange/diafiltration, the calculated step yield for monomeric antibody was approximately 86 %. The "monomer peak" of the retentate detected by SE-HPLC was 65%. The single stage TFF slightly increased the HCP load of the product stream by 6% and the HCDNA load was reduced by 17%. This is the identical unit operation as the single stage TFF reported in Example 3.

Following the second stage TFF with a 300 kD cut off, the calculated step yield for monomeric antibody was 100%. The "monomer peak" detected by SE-HPLC was 75%, which is significantly higher than that of the applied feed (65%). The second stage TFF reduced the HCP load and HCDNA load of the product stream by 29% and 86%, respectively.

For the subsequent TFF/concentration step using a column with a 50 kD cut-off, the calculated step yield (monomeric antibody) was 100%. For the eluted ample, the "monomer peak" detected by SE-HPLC was 76%, which was slightly higher that that in the applied feed sample. The TFF/concentration stage increased the HCP load of the product stream by 10%, while the HCDNA load was reduced by 3%.

AEX subsequent to dual stage TFF peconditioning was operated in flow through mode. The calculated step yield for the AEX (monomeric antibody) was 90%. The "monomer peak" detected by SE-HPLC was 80%, slightly higher compared with the feed sample (76%). The AEX unit operation reduced HCP load of the product stream by 64%, while the HCDNA load was reduced by 87%. In contrast to AEX using a single stage TFF processing as in Example 3 (and Example 1), the HCP and HCDNA data confirm the results of Example 2, namely that AEX subsequent to the dual stage TFF processing significantly contributes to the purification process, even though it is operated in flow-through mode at comparably high column loading.

Prior to the CEX step, the pH of the product stream was adjusted to 5.0. Again, in contrast with the single stage TFF processing reported in Examples 1 and 3, and consistent with the dual stage TFF processing reported in Example 2, no precipitation was observed in the product stream at this stage, and the sample was loaded directly onto the CEX column.

The CEX column was operated in bind-and-elute mode. The calculated step yield (monomeric antibody) was 100%. For the product, the "monomer peak" detected by SE-HPLC was 95%, which was much higher compared to that in the feed sample (80%). CEX reduced the HCP and HCDNA load of the feed sample by 93% and 99.8%, respectively. The removal of antibody aggregates and fragments, as well as the dramatic reduction of HCP and HCDNA, demonstrate that CEX efficiently removes the remaining impurities at a column loading of 20 g/l (consistent with Example 2).

### General Conclusions In View Of The Results OF Examples 1 To 4

Using a single stage TFF method according to methods known in the art failed to satisfactorily reduce contaminant levels (*e.g.,* HCP and HCDNA), resulting in the overloading of downstream chromatographic process, *e.g.,* AEX. However, by employing a dual stage TFF method according to the present invention, overloading of the AEX column was prevented at a given column size. This indicates that the dual stage TFF methods allow efficient contaminant reduction, *e.g.,* HCDNA and/or HCP removal, for the same initial feed stream using significantly smaller chromatographic columns than are currently implemented. This represents a significant economic advantage associated with the dual stage TFF method disclosed herein.

The state of the art demonstrates the interest in using non-affinity based purification, *e.g.,* AEX and CEX. However, these methods have been unsatisfactory and are not currently viewed a meaningful replacement for affinity based systems. In particular, the implementation of non-affinity chromatographic processes is often associated with undesired effects such as precipitate formation in the product stream. For example, product stream processing downstream of a single stage TFF to adjust the pH from 7.5 to 5.0 prior to the CEX resulted in impurity precipitation. Processing of the product stream to remove precipitate is time consuming and otherwise associated with significant cost as detailed herein. However, implementation of the dual stage TFF methods of the invention eliminated precipitate formation during this processing step, increasing yields and saving on the costs associated with precipitate processing.

Direct comparison of the single stage TFF and dual stage TFF of the invention revealed that the latter is superior with respect to product quality with respect to the level of monomeric product, HCP level and HCDNA level.

## Claims

1. A method of separating a protein of interest from a cell culture solution containing said protein using dual stage tangential-flow ultrafiltration (TFF), wherein said dual stage TFF comprises a first TFF unit operation and a second TFF unit operation, wherein
said first TFF unit operation filters a first product stream containing said protein using a first ultrafiltration membrane having a cut-off value such that said protein is recovered in the retentate of the first TFF unit operation; and
said second TFF unit operation filters a second product stream containing said protein using a second ultrafiltration membrane having a cut-off value such that said protein is recovered in the permeate of the second TFF unit operation;
and wherein said first or said second product stream is a cell culture supernatant.

2. The method according to claim 1, wherein said first product stream is a cell culture supernatant and said first TFF unit operation is upstream of said second TFF unit operation.

3. The method according to claim 1 or 2, wherein said dual stage TFF further comprises a third TFF unit operation that filters a third product stream containing said protein using a third ultrafiltration membrane having a cut-off value such that said protein is in the retentate of the third TFF unit operation, and wherein said third TFF unit operation is downstream of said first and second TFF unit operations.

4. The method according to claim 3, wherein the cut-off value of the third ultrafiltration membrane is the same as the cut-off value of the first ultrafiltration membrane.

5. The method according to any one of the preceding claims, wherein said protein has a molecular weight of at least 10 kD but not greater than 500 kD.

6. The method according to claim 5,
wherein said first ultrafiltration membrane has a cut-off value of one-half (0.5 times) the molecular weight of said protein or less; and
wherein said second ultrafiltration membrane has a cut-off value of at least twice (2 times) the molecular weight of said protein but not greater than 1000 kD.

7. The method according to claim 5 or 6, wherein said protein has a molecular weight of 150 ± 75 kD.

8. The method according to any one of the preceding claims, wherein said protein is an antibody.

9. The method according to any one of claims 5 to 8, wherein said first ultrafiltration membrane has a cut-off value of 50 kD or less; and wherein said second ultrafiltration membrane has a cut-off value of between 300 kD and 1000 kD.

10. The method according to claim 9, wherein said first ultrafiltration membrane has a cut-off value of 50 kD; and wherein said second ultrafiltration membrane has a cut-off value of 300 kD.

11. The method according to any one of the preceding claims, wherein said dual-stage TFF reduced the concentration of host cell protein (HCP) in the solution containing the protein of interest by at least 10% relative to said cell culture supernatant and/or reduces the concentration of host cell DNA (HCDNA) in the solution containing the protein of interest by at least 30% relative to said cell culture supernatant.

12. The method according to any one of claims 8 to 10, wherein said dual-stage TFF reduces the concentration of HCP in the solution containing the protein of interest to 400,000 ng per mg of said protein or less and/or reduces the concentration of HCDNA in the solution containing the protein of interest to 1,500,000 pg per mg of said protein or less.

13. The method according to any one of the preceding claims, further comprising a column chromatography process downstream of said dual-stage TFF.

14. The method according to claim 13, wherein said column chromatography process is a non-affinity column chromatography process.

15. The method according to claim 13 or 14, wherein said column chromatography process is an anion exchange chromatography (AEX) unit operation or a cation exchange chromatography (CEX) unit operation.

## Patentansprüche

1. Verfahren zum Trennen eines Proteins von Interesse aus einer Zellkulturlösung, die das Protein enthält, unter Verwendung einer zweistufigen Tangentialflussfiltration (TFF), wobei die zweistufige TFF einen ersten TFF-Einheit-Vorgang und einen zweiten TFF-Einheit-Vorgang umfasst, wobei
der Vorgang der ersten TFF-Einheit einen ersten Produktstrom filtert, der das Protein enthält, unter Verwendung einer ersten Ultrafiltrationsmembran, die einen Cut-off-Wert hat, so dass das Protein im Retentat des ersten TFF-Einheit-Vorgangs gewonnen wird; und
der Vorgang der zweiten TFF-Einheit einen zweiten Produktstrom filtert, der das Protein enthält, unter Verwendung einer zweiten Ultrafiltrationsmembran, die einen Cut-off-Wert hat, so dass das Protein im Permeat des zweiten TFF-Einheit-Vorgangs gewonnen wird;
und wobei der erste oder der zweite Produktstrom ein Zellkulturüberstand ist.

2. Verfahren nach Anspruch 1, wobei der erste Produktstrom ein Zellkulturüberstand und der erste TFF-Einheit-Vorgang stromaufwärts des zweiten TFF-Einheit-Vorgangs ist.

3. Verfahren nach Anspruch 1 oder 2, wobei die zweistufige TFF des Weiteren einen dritten TFF-Einheit-Vorgang umfasst, der einen dritten Produktstrom filtert, der das Protein enthält, unter Verwendung einer dritten Ultrafiltrationsmembran, die einen Cut-off-Wert hat, so dass das Protein im Retentat des dritten TFF-Einheit-Vorgangs ist, und wobei der dritte TFF-Einheit-Vorgang stromabwärts der ersten und zweiten TFF-Einheit-Vorgänge ist.

4. Verfahren nach Anspruch 3, wobei der Cut-off-Wert der dritten Ultrafiltrationsmembran der gleiche ist wie der Cut-off-Wert der ersten Ultrafiltrationsmembran.

5. Verfahren nach einem der vorangehenden Ansprüche, wobei das Protein ein Molekulargewicht von mindestens 10 kD aber nicht größer als 500 kD hat.

6. Verfahren nach Anspruch 5,
wobei die erste Ultrafiltrationsmembran einen Cut-off-Wert von der Hälfte (mal 0,5) des Molekulargewichts des Proteins oder weniger hat; und
wobei die zweite Ultrafiltrationsmembran einen Cut-off-Wert von mindestens des Doppelten (mal zwei) des Molekulargewichts des Proteins aber nicht größer als 1000 kD hat.

7. Verfahren nach Anspruch 5 oder 6, wobei das Protein ein Molekulargewicht von 150 ± 75 kD hat.

8. Verfahren nach einem der vorangehenden Ansprüche, wobei das Protein ein Antikörper ist.

9. Verfahren nach einem der Ansprüche 5 bis 8, wobei die erste Ultrafiltrationsmembran einen Cut-off-Wert von 50 kD oder weniger hat; und wobei die zweite Ultrafiltrationsmembran einen Cut-off-Wert von zwischen 300 kD und 1000 kD hat.

10. Verfahren nach Anspruch 9, wobei die erste Ultrafiltrationsmembran einen Cut-off-Wert von 50 kD hat; und wobei die zweite Ultrafiltrationsmembran einen Cut-off-Wert von 300 kD hat.

11. Verfahren nach einem der vorangehenden Ansprüche, wobei die zweistufige TFF die Konzentration des Wirtszellproteins (host cell protein; HCP) in der Lösung, die das Protein von Interesse enthält, um mindestens 10% reduziert im Bezug auf den Zellkulturüberstand und/oder die Konzentration der Wirtzell-DNA (host cell DNA; HCDNA) in der Lösung reduziert, die das Protein von Interesse enthält um mindestens 30% reduziert im Bezug auf den Zellkulturüberstand.

12. Verfahren nach einem der Ansprüche 8 bis 10, wobei die zweistufige TFF die Konzentration des HCP in der Lösung, die das Protein von Interesse enthält, auf 400.000 ng pro mg des Proteins oder weniger reduziert und/oder die Konzentration der HCDNA in der Lösung, die das Protein von Interesse enthält, auf 1.500.000 pg pro mg des Proteins oder weniger reduziert.

13. Verfahren nach einem der vorangehenden Ansprüche, des Weiteren umfassend einen Säulenchromatographie-Prozess stromabwärts der zweistufigen TFF.

14. Verfahren nach Anspruch 13, wobei der Säulenchromatographie-Prozess ein Nicht-Affinitäts-Säulenchromatographie-Prozess ist.

15. Verfahren nach Anspruch 13 oder 14, wobei der Säulenchromatographie-Prozess ein Anion-Austausch-Chromatographie (anion exchange chromatography; AEX)-Einheit-Vorgang oder ein Cation-Austausch-Chromatographie (cation exchange chromatography; CEX)-Einheit-Vorgang ist.

## Revendications

1. Procédé de séparation d'une protéine d'intérêt à partir d'une solution de culture cellulaire contenant ladite protéine en utilisant l'ultrafiltration à flux tangentiel (TFF) en deux étapes, dans lequel ladite TFF en deux étapes comprend une première opération unitaire de TFF et une seconde opération unitaire de TFF, dans lequel
ladite première opération unitaire de TFF filtre un premier courant de produit contenant ladite protéine en utilisant une première membrane d'ultrafiltration ayant une valeur seuil telle que ladite protéine est récupérée dans le rétentat de la première opération unitaire de TFF ; et
ladite seconde opération unitaire de TFF filtre un second courant de produit contenant ladite protéine en utilisant une seconde membrane d'ultrafiltration ayant une valeur seuil telle que ladite protéine est récupérée dans le perméat de la seconde opération unitaire de TFF ;
et dans lequel ledit premier ou ledit second courant de produit est un surnageant de culture cellulaire.

2. Procédé selon la revendication 1, dans lequel ledit premier courant de produit est un surnageant de culture cellulaire et ladite première opération unitaire de TFF est en amont de ladite seconde opération unitaire de TFF.

3. Procédé selon la revendication 1 ou 2, dans lequel ladite TFF en deux étapes comprend en outre une troisième opération unitaire de TFF qui filtre un troisième courant de produit contenant ladite protéine en utilisant une troisième membrane d'ultrafiltration ayant une valeur seuil telle que ladite protéine est dans le rétentat de la troisième opération unitaire de TFF, et dans lequel ladite troisième opération unitaire de TFF est en aval desdites première et deuxième opérations unitaires de TFF.

4. Procédé selon la revendication 3, dans lequel la valeur seuil de la troisième membrane d'ultrafiltration est identique à la valeur seuil de la première membrane d'ultrafiltration.

5. Procédé selon l'une quelconque des revendications précédentes, dans lequel ladite protéine a une masse moléculaire en poids d'au moins 10 kD mais pas supérieure à 500 kD.

6. Procédé selon la revendication 5,
dans lequel ladite première membrane d'ultrafiltration a une valeur seuil de la moitié (0,5 fois) de la masse moléculaire en poids de ladite protéine ou inférieure ; et
dans lequel ladite seconde membrane d'ultrafiltration a une valeur seuil d'au moins deux fois (2 fois) la masse moléculaire en poids de ladite protéine mais pas supérieure à 1000 kD.

7. Procédé selon la revendication 5 ou 6, dans lequel ladite protéine a une masse moléculaire en poids de 150 ± 75 kD.

8. Procédé selon l'une quelconque des revendications précédentes, dans lequel ladite protéine est un anticorps.

9. Procédé selon l'une quelconque des revendications 5 à 8, dans lequel ladite première membrane d'ultrafiltration a une valeur seuil de 50 kD ou inférieure ; et dans lequel ladite seconde membrane d'ultrafiltration a une valeur seuil située entre 300 kD et 1000 kD.

10. Procédé selon la revendication 9, dans lequel ladite première membrane d'ultrafiltration a une valeur seuil de 50 kD ; et dans lequel ladite seconde membrane d'ultrafiltration a une valeur seuil de 300 kD.

11. Procédé selon l'une quelconque des revendications précédentes, dans lequel ladite TFF en deux étapes a réduit la concentration des protéines de la cellule hôte (HCP) dans la solution contenant la protéine d'intérêt d'au moins 10 % par rapport au dit surnageant de culture cellulaire et/ou réduit la concentration de l'ADN de la cellule hôte (HCDNA) dans la solution contenant la protéine d'intérêt d'au moins 30 % par rapport au dit surnageant de culture cellulaire.

12. Procédé selon l'une quelconque des revendications 8 à 10, dans lequel ladite TFF en deux étapes réduit la concentration des HCP dans la solution contenant la protéine d'intérêt à 400 000 ng par mg de ladite protéine ou moins et/ou réduit la concentration de HCDNA dans la solution contenant la protéine d'intérêt à 1 500 000 pg par mg de ladite protéine ou moins.

13. Procédé selon l'une quelconque des revendications précédentes, comprenant en outre un procédé de chromatographie sur colonne en aval de ladite TFF en deux étapes.

14. Procédé selon la revendication 13, dans lequel ledit procédé de chromatographie sur colonne est un procédé de chromatographie sur colonne non d'affinité.

15. Procédé selon la revendication 13 ou 14, dans lequel ledit procédé de chromatographie sur colonne est une opération unitaire de chromatographie d'échange d'anions (AEX) ou une opération unitaire de chromatographie d'échange de cations (CEX).
